# EUROPEAN PATENT APPLICATION

(11) **EP 4 678 745 A1**
(43) Date of publication of application: **14.01.2026**
(21) Application number: 24767254.6
(22) Date of filing: 08.03.2024
(51) Int. Cl.: C12N 15/113, C12N 9/22, C12N 15/55, C12N 15/63

(54) **GUIDE RNA AND USE METHOD THEREOF**

(30) Priority: 09.03.2023 US 202363450962 P; 09.03.2023 US 202363450967 P
(71) Applicant: Kyushu University, National University Corporation, Nishi-ku Fukuoka-shi Fukuoka 819-0395 (JP)
(72) Inventor: SUZUKI, Atsushi, Fukuoka-shi, Fukuoka 819-0395 (JP); KAWAMATA, Masaki, Fukuoka-shi, Fukuoka 819-0395 (JP)
(74) Representative: EP&C
(86) International application number: PCT/JP2024/009107
(87) International publication number: WO 2024/185889

(57) **Abstract**

Provided is a method for controlling transcription of RNA, including: a step of transcribing RNA from an expression vector for the RNA, in which the RNA is RNA to which one or more nucleotide residues are added at the 5' end, and the like. In addition, provided is a method for designing an expression vector for RNA, including: designing an expression vector for RNA in which transcription efficiency of the RNA is controlled by adjusting one or more selected from the group consisting of the number of nucleotide residues to be added to the 5' end of an RNA coding sequence and the type of the nucleotide residues, and the like.

## Description

### TECHNICAL FIELD

The present invention relates to guide RNA and a use method thereof. In particular, the present invention relates to a method for controlling transcription of RNA, a method for designing an expression vector for RNA, a method for controlling transcription of a target gene, a genome editing method, a base editing method, a kit for controlling transcription of a target gene, a base editing kit, a genome editing kit, guide RNA, and an expression vector.

Priority is claimed on U.S. Provisional Application Nos. 63/450,962 and 63/450,967 filed in the United States on March 9, 2023, the contents of which are incorporated herein by reference.

### BACKGROUND ART

Clustered Regularly Interspaced Short Palindromic Repeats (CRISPR), together with CRISPR-associated (Cas) genes, are known to constitute an adaptive immune system in bacteria and archaea that provides acquired resistance against invading foreign nucleic acids. CRISPR often originates from phage or plasmid DNA and consists of short, conserved repeat sequences of 24 to 48 bp, interspersed with unique variable DNA sequences called spacers, which are similar in size. In the vicinity of the repeat sequences and spacer sequences, there exists a group of genes encoding the Cas protein family.

In the CRISPR/Cas system, foreign DNA is cleaved into fragments of approximately 30 bp by the Cas protein family and inserted into CRISPR. The Cas1 and Cas2 proteins, which are members of the Cas protein family, recognize a nucleotide sequence called a protospacer adjacent motif (PAM) in the foreign DNA, cleave its upstream region, and insert it into the host's CRISPR sequence, which becomes the bacterial immune memory. The CRISPR sequence, including the immune memory, is transcribed to produce RNA (pre-crRNA), which pairs with partially complementary RNA (trans-activating crRNA: tracrRNA) and is incorporated into the Cas9 protein, a member of the Cas protein family. The pre-crRNA and tracrRNA incorporated into Cas9 are cleaved by RNAase III, forming small RNA fragments (CRISPR-RNAs: crRNAs) containing the foreign sequence (guide sequence), and a Cas9-crRNA-tracrRNA complex is formed. The Cas9-crRNA-tracrRNA complex binds to foreign invasive DNA complementary to the crRNA, and the Cas9 protein, which is an enzyme (nuclease) that cleaves DNA, cleaves the foreign invasive DNA, thereby suppressing and eliminating the function of the DNA invading from the outside.

The Cas9 protein recognizes the PAM sequence in foreign invasive DNA and cleaves the double-stranded DNA upstream thereof to produce blunt ends. The length and nucleotide sequence of the PAM sequence vary depending on the bacterial species, and in *Streptococcus pyogenes (S. pyogenes),* it recognizes the three bases "NGG." *Streptococcus thermophilus (S. thermophilus)* has two Cas9 proteins, which recognize the five to six bases "NGGNG" or "NNAGAA" as the PAM sequence, respectively (N represents any base). The position of cleavage, located a few bp upstream of the PAM sequence, also varies depending on the bacterial species, but most Cas9 orthologs, including *S. pyogenes,* cleave three bases upstream of the PAM sequence.

In recent years, technologies applying the CRISPR/Cas system in bacteria to genome editing have been actively developed. A construct in which crRNA and tracrRNA are fused and expressed as a tracrRNA-crRNA chimera (sgRNA) has been utilized. The sgRNA recruits an RNA-guided nuclease (RGN), which cleaves the genomic DNA at the target site.

Types I to VI of the CRISPR/Cas system are currently known. The CRISPR/Cas system predominantly used for genome editing is the Type II CRISPR/Cas system, where the Cas9 protein is a Type II RGN, and Cas12a (Cpf1) is a Type V RGN. The Cas9 protein derived from *Streptococcus pyogenes* recognizes the three bases NGG as the PAM sequence, allowing it to cleave upstream of any sequence with two consecutive guanines.

In recent years, various technologies utilizing the CRISPR/Cas system have been developed. For example, Patent Document 1 describes a method for improving the genome editing efficiency in one allele by adding nucleotide residues to the 5' end of the spacer sequence.

### Citation List

### Patent Document

Patent Document 1: PCT International Publication No. WO2020/122195

### SUMMARY OF INVENTION

### Technical Problem

Further technological development regarding the control of RNA transcription is desired. In addition, further technological development applying RNA-guided nucleases is desired.

An object of the present disclosure is to provide novel methods relating to a method for controlling transcription of RNA and a method for designing an expression vector for RNA, as well as novel methods applicable to application technologies of RNA-guided nucleases. Another object is to provide guide RNA, expression vectors, kits, and the like usable in the above-described methods.

### Solution to Problem

The present disclosure has the following aspects.
[1] A method for controlling transcription of RNA, including: a step of transcribing RNA from an expression vector for the RNA, in which the RNA is RNA to which one or more nucleotide residues are added at the 5' end.
[2] The method for controlling transcription of RNA according to [1], in which the number of the added nucleotide residues is three or more.
[3] The method for controlling transcription of RNA according to [1] or [2], in which the RNA is guide RNA.
[4] A method for designing an expression vector for RNA, including: designing an expression vector for RNA in which transcription efficiency of the RNA is controlled by adjusting one or more selected from the group consisting of the number of nucleotide residues to be added to the 5' end of an RNA coding sequence and the type of the nucleotide residues.
[5] The method for designing an expression vector for RNA according to [4], in which the RNA is guide RNA.
[6] A method for controlling transcription of a target gene, including: a step of introducing, into a cell, (A) at least one selected from the group consisting of (a1) guide RNA to which one or more nucleotide residues are added at the 5' end, and (a2) an expression vector for the guide RNA of (al); and (B) at least one selected from the group consisting of a fusion protein including an RNA-guided nuclease with inactivated nuclease activity and a transcription regulation-related domain, mRNA of the fusion protein, and an expression vector for the fusion protein, in which the guide RNA targets a transcription regulation region of the target gene.
[7] The method for controlling transcription of a target gene according to [6], in which the number of the added nucleotide residues is three or more.
[8] A genome editing method, including: a step of introducing, into a cell, (A) at least one selected from the group consisting of (a1) guide RNA which has a repeat sequence on the 5' side of a spacer sequence and to which one or more nucleotide residues are added at the 5' end, and (a2) an expression vector for the guide RNA of (al); and (B) at least one selected from the group consisting of an RNA-guided nuclease, mRNA of the RNA-guided nuclease, and an expression vector for the RNA-guided nuclease.
[9] The genome editing method according to [8], in which the number of the added nucleotide residues is three or more.
[10] The genome editing method according to [8] or [9], in which the RNA-guided nuclease is Cas protein of a Type V CRISPR/Cas system.
[11] A base editing method, including: a step of introducing, into a cell, (A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (a1); and (B) at least one selected from the group consisting of a base editor, mRNA of the base editor, and an expression vector for the base editor.
[12] The base editing method according to [11], in which an activity window of the base editor is controlled by adjusting one or more selected from the group consisting of (1) the addition of nucleotide residues to the 5' end, (2) the length of a spacer sequence, and (3) the introduction of mismatched nucleotide residues in the spacer sequence.
[13] The base editing method according to [12], in which the activity window of the base editor is controlled by adjusting the addition of nucleotide residues to the 5' end, and the number of the added nucleotide residues is three or more.
[14] The base editing method according to [12], in which the activity window of the base editor is controlled by adjusting the length of a spacer sequence.
[15] The base editing method according to [12], in which the activity window of the base editor is controlled by adjusting the introduction of mismatched nucleotide residues in the spacer sequence.
[16] A kit for controlling transcription of a target gene, including: at least one selected from the group consisting of (a1) guide RNA to which one or more nucleotide residues are added at the 5' end, and (a2) an expression vector for the guide RNA of (al); and (B) at least one selected from the group consisting of a fusion protein including an RNA-guided nuclease with inactivated nuclease activity and a transcription regulation-related domain, mRNA of the fusion protein, and an expression vector for the fusion protein, in which the guide RNA targets a transcription regulation region of the target gene.
[17] A base editing kit, including: (A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (al); and (B) at least one selected from the group consisting of a base editor, mRNA of the base editor, and an expression vector for the base editor.
[18] An expression vector capable of expressing: (A) guide RNA to which one or more nucleotide residues are added at the 5' end; and (B) a fusion protein including an RNA-guided nuclease with inactivated nuclease activity and a transcription regulation-related protein.
[19] An expression vector capable of expressing: (A) guide RNA to which one or more nucleotide residues are added at the 5' end; and (B) a base editor.
[20] Guide RNA which has a repeat sequence on the 5' side of a spacer sequence and to which one or more nucleotide residues are added at the 5' end.
[21] An expression vector capable of expressing the guide RNA according to [20].
[22] The expression vector according to [21], further capable of expressing an RNA-guided nuclease.
[23] A genome editing kit, including: (A) at least one selected from the group consisting of (a1) guide RNA which has a repeat sequence on the 5' side of a spacer sequence and to which one or more nucleotide residues are added at the 5' end, and (a2) an expression vector for the guide RNA of (a1); and (B) at least one selected from the group consisting of an RNA-guided nuclease, mRNA of the RNA-guided nuclease, and an expression vector for the RNA-guided nuclease.

### Advantageous Effects of Invention

According to the present disclosure, it is possible to provide novel methods relating to a method for controlling transcription of RNA and a method for designing an expression vector for RNA, as well as novel methods applicable to application technologies of RNA-guided nucleases. In addition, it is possible to provide guide RNA, expression vectors, kits, and the like usable in the above-described methods.

### BRIEF DESCRIPTION OF DRAWINGS

[FIG. 1] A schematic diagram illustrating the structure of guide RNA (gRNA) having added nucleotide residues in one embodiment.
[FIG. 2] A schematic diagram illustrating the AIMS cells used in examples. "MP" represents a membrane protein. "TF" represents a transcription regulatory factor.
[FIG. 3] A diagram showing the results of Northern blot analysis in Experimental Example 1. "Trim" represents gRNA in which the added nucleotide residues have been trimmed.
[FIG. 4] The quantified results of the signal from the Northern blot analysis in FIG. 3.
[FIG. 5] The results of the Northern blot analysis in Experimental Example 2. "15hp" represents a sample using sgRNA having added nucleotide residues of 15 nucleotide residues and forming a hairpin structure. "15s" represents a sample using sgRNA having 5'-added nucleotide residues of 15 nucleotide residues and not forming a hairpin structure.
[FIG. 6] The results of a transcription activation test by CRISPRa in Experimental Example 3.
[FIG. 7] The results of the transcription activation test by CRISPRa in Experimental Example 3.
[FIG. 8] The results of a transcription suppression test by CRISPRi in Experimental Example 4.
[FIG. 9] The results of an indel induction test using Cas12a (AsCpf1) in Experimental Example 5. The results examine the introduction position of added nucleotide residues in gRNA. In the schematic diagram of gRNA shown below the horizontal axis of the graph, [C] indicates the position where the added nucleotide residues are introduced. "No" indicates cases where no indel was induced. "Mono" indicates cases where an indel was induced in one allele. "Bi" indicates cases where indels were induced in both alleles.
[FIG. 10] The results of the indel induction test using Cas12a (AsCpf1) in Experimental Example 5. The results examine the number of added nucleotide residues in gRNA.
[FIG. 11] The results of clone sequence analysis of a base substitution test using ABE8e in Experimental Example 6.
[FIG. 12] The results of clone sequence analysis of a base substitution test using ABE8e in Experimental Example 7.
[FIG. 13] The results of amplicon sequence analysis of a base substitution test using ABE8e in Experimental Example 8.
[FIG. 14] The results of examining the indel induction rate from the amplicon sequence analysis results of the base substitution test using ABE8e conducted in Experimental Example 8, in Experimental Example 9.
[FIG. 15] The results of amplicon sequence analysis of a base substitution test using ABE8e in Experimental Example 10.
[FIG. 16] The value (Off/On ratio) obtained by dividing the "Editing frequency (%)" of "A₅G₆--A_{g}>G₅G₆--G_{g}" in FIG. 15 by the "Editing frequency (%)" of "A₅A₆-A₉>G₅G₆--G₉" in FIG. 13.
[FIG. 17] The results of amplicon sequence analysis of a base substitution test using ABE8e in Experimental Example 11. The results show the analysis of the On-target region.
[FIG. 18] The results of the amplicon sequence analysis of the base substitution test using ABE8e in Experimental Example 11. The results show the analysis of the Off-target 1 region (upper figure) and the Off-target 2 region (lower figure). Nucleotide residues enclosed in a square indicate mismatched residues.
[FIG. 19] The value (Off/On ratio) obtained by dividing the editing frequency in the Off-target 1 region (upper figure) or the Off-target 2 region (lower figure) in Experimental Example 11 by the editing frequency in the On-target.
[FIG. 20] The target genome site of an SNP repair test for the Acrv gene related to fibrodysplasia ossificans progressiva (FOP) in Experimental Example 12. Byproducts (1) to (3) indicate the predicted mechanism by which byproduct correction occurs.
[FIG. 21] The results of the FOP-related SNP repair test in Experimental Example 12.
[FIG. 22] The target genome site of a hereditary tyrosinemia type I (HT-1)-related SNP repair test in Example 13.
[FIG. 23] The results of the HT-1-related SNP repair test in Experimental Example 13.
[FIG. 24] The structure of the ABE8e all-in-one plasmid used in Experimental Example 14.
[FIG. 25] The results of an HT-1-related SNP repair test in Experimental Example 14.
[FIG. 26] The results of an amplicon sequence analysis of a base substitution test using ABE8e in Experimental Example 15.
[FIG. 27] The results of the amplicon sequence analysis of the base substitution test using ABE8e in Experimental Example 15.
[FIG. 28] The results of an amplicon sequence analysis of a base substitution test using AID-BE4max in Experimental Example 16.
[FIG. 29] The results of the amplicon sequence analysis of the base substitution test using AID-BE4max in Experimental Example 16.

### DESCRIPTION OF EMBODIMENTS

### [Definition]

The numerical range represented by "to" means a range that includes the numerical values denoted before and after "to" as a lower limit value and an upper limit value, respectively.

The term "comprise" means that constituent elements in addition to target constituent elements are included. The term "consist of" means that constituent elements in addition to target constituent elements are not included. The term "consist essentially of" means that constituent elements in addition to target constituent elements are not included in an aspect exhibiting a special function (such as an aspect in which the effect of the invention is completely lost). In a case where the term "comprise" is mentioned in the present specification, the aspects of "consist of" and "consist essentially of" are also included.

Proteins, nucleic acids (DNA, RNA), vectors, and cells may be isolated. The term "isolated" means a state separated from a natural state or other components. An "isolated" component may be substantially free of other components. The phrase "substantially free of other components" means that the amount of other components in the isolated component is negligible. The amount of other components in the isolated component may be, for example, 10 mass% or less, 5 mass% or less, 4 mass% or less, 3 mass% or less, 2 mass% or less, 1 mass% or less, 0.5 mass% or less, or 0.1 mass% or less. The proteins, nucleic acids (DNA, RNA), vectors, and cells described in the present specification may be an isolated protein, an isolated nucleic acid (isolated DNA, isolated RNA), an isolated vector, and an isolated cell, respectively.

The terms "polynucleotide" and "nucleic acid" are used interchangeably and refer to a nucleotide polymer in which nucleotides are linked by phosphodiester bonds. A "polynucleotide" or "nucleic acid" may be DNA, RNA, or a combination of DNA and RNA. A "polynucleotide" or "nucleic acid" may be a polymer of natural nucleotides, a polymer of natural and non-natural nucleotides, or a polymer of non-natural nucleotides. Non-natural nucleotides are analogs of natural nucleotides. A non-natural nucleotide is a nucleotide in which at least one of the base moiety, sugar moiety, or phosphate moiety of a natural nucleotide is modified (for example, a nucleotide having a phosphorothioate backbone).

A nucleotide sequence of a "polynucleotide" or "nucleic acid" is described using the generally accepted one-letter code unless otherwise specified. Unless otherwise specified, the nucleotide sequence is described from the 5' side to the 3' side. The nucleotide residues constituting a "polynucleotide" or "nucleic acid" may be described simply as adenine, thymine, cytosine, guanine, uracil, or the like, or by their one-letter codes.

A "gene" refers to a polynucleotide that includes at least one open reading frame encoding a specific protein. A gene may include both exons and introns.

The terms "polypeptide," "peptide," and "protein" are used interchangeably and refer to a polymer of amino acids linked by amide bonds. A "polypeptide," "peptide," or "protein" may be a polymer of natural amino acids, a polymer of natural and non-natural amino acids, or a polymer of non-natural amino acids. Non-natural amino acids are analogs of natural amino acids. Non-natural amino acids are, for example, amino acids having a side chain other than the side chain of a natural amino acid. Unless otherwise specified, an amino acid sequence is described from the N-terminal side to the C-terminal side.

The term "allele" refers to a pair of genes located at the same locus on a pair of chromosomes or a pair of nucleotide sequences located at the same locus. The pair of genes does not necessarily need to be allelic, and the pair of nucleotide sequences does not necessarily need to be different nucleotide sequences. The term "both alleles" refers to both of the pair of genes or both of the pair of nucleotide sequences. The term "one allele" refers to either one of the pair of genes or either one of the pair of nucleotide sequences.

The term "genome editing" refers to inducing a mutation at a desired position (target region) of a genome. Genome editing may include the use of a nuclease engineered to cleave DNA at the target region. Typically, the use of a site-specific nuclease induces a double-strand break (DSB) in the DNA at the target region. Subsequently, the genome is repaired by an endogenous cellular process, such as homologous directed repair (HDR) or non-homologous end-joining non-homologous end-joining repair (NHEJ). NHEJ is a repair method that ligates the ends of a double-strand break without using a repair template DNA, frequently inducing insertions and/or deletions (indels) during repair. HDR is a repair mechanism that uses a repair template DNA, enabling the introduction of a desired mutation into the target region. In some embodiments, an RNA-guided nuclease is used in genome editing technology. In some embodiments, a CRISPR/Cas system is used in the genome editing technology.

The term "repair template DNA" refers to DNA used for repairing a double-strand break in DNA, which is capable of homologous recombination with DNA surrounding a target region. "Repair template DNA" is also referred to as "donor DNA." In some embodiments, donor DNA may include a nucleotide sequence having 95% or greater sequence identity with a target region and its surrounding regions. Donor DNA may include homology arms capable of homologous recombination with regions surrounding the target region. Homology arms may consist of a 5' homology arm and a 3' homology arm. For example, relative to a DNA strand containing a target sequence, a homology arm containing DNA homologous to a region located on the 5' side of the target sequence may be referred to as a 5' homology arm, and a homology arm containing DNA homologous to a region located on the 3' side of the target sequence may be referred to as a 3' homology arm. A desired nucleotide sequence may be included between the 5' homology arm and the 3' homology arm. The length of the 5' homology arm and the 3' homology arm may be any length sufficient to allow homologous recombination to occur. Examples of the lower limit value of the length of the 5' homology arm and the 3' homology arm include 30 bp or more, 40 bp or more, 50 bp or more, 100 bp or more, 200 bp or more, 300 bp or more, 400 bp or more, and 500 bp or more, respectively. The upper limit of the length of the 5' homology arm and the 3' homology arm is not particularly limited but may be, for example, 10 kb or less. Examples of the length of the 5' homology arm and the 3' homology arm include 30 bp to 10 kb, 40 bp to 10 kb, 50 bp to 10 kb, 100 bp to 10 kb, 200 bp to 10 kb, 300 bp to 10 kb, 400 bp to 10 kb, and 500 bp to 10 kb, respectively. The length of the 5' homology arm and the 3' homology arm may be the same or different from each other. The term "homologous DNA" refers to, for example, DNA having 80% or greater, preferably 85% or greater, more preferably 90% or greater, and still more preferably 95% or greater sequence identity with a region subject to homologous recombination.

The term "safe harbor region" refers to a region on a genome where insertion of foreign DNA has been demonstrated to be possible without causing harmful effects to a cell. As safe harbor regions, AAVS1 in humans and Rosa26 in mice are known, for example.

The term "RNA-guided nuclease" refers to an enzyme that collaborates with guide RNA and exhibits sequence-specific nuclease activity induced by the guide RNA. An RNA-guided nuclease forms a complex with guide RNA and exhibits activity to cleave a nucleic acid near a target sequence bound by the guide RNA. Examples of RNA-guided nucleases include Cas protein. RNA-guided nucleases also include RNA-guided nucleases with inactivated nuclease activity. Examples of RNA-guided nucleases with inactivated nuclease activity include RNA-guided nucleases having no nuclease activity but having nickase activity, and RNA-guided nucleases having neither nuclease activity nor nickase activity.

The term "Cas protein" refers to a CRISPR-associated protein. In some embodiments, Cas protein forms a complex with guide RNA and has endonuclease activity or nickase activity. Cas protein may be any Cas protein of Type I, Type II, Type III, Type IV, Type V, or Type VI CRISPR/Cas systems. Examples of Cas proteins include, but are not limited to, Cas3 protein, Cas9 protein, Cas12a (Cpf1) protein, C2c1 protein, C2c2 protein, and C2c3 protein. Cas protein may be any of a wild-type Cas protein, its homologs (paralogs, orthologs, or the like), or variants thereof, as long as it has the ability to form a complex with guide RNA. Cas protein also includes Cas protein having no nuclease activity. Examples of Cas proteins having no nuclease activity include Cas protein having no nuclease activity but having nickase activity, and Cas protein having neither nuclease activity nor nickase activity. Cas protein having no nuclease activity can be obtained by mutating one or more amino acid residues of wild-type Cas protein having nuclease activity. Cas protein having nickase activity can be obtained, for example, by mutating one or more amino acid residues of a wild-type Cas protein having nuclease activity. These can be obtained by known methods.

In some embodiments, Cas protein is involved in a Class 2 CRISPR/Cas system. Preferably, Cas protein is involved in a Type II CRISPR/Cas system or a Type V CRISPR/Cas system. Examples of Cas proteins involved in a Type II CRISPR/Cas system include Cas9 protein. Examples of Cas proteins involved in a Type V CRISPR/Cas system include Cas12a protein. Cas12a protein is also referred to as Cpf1 protein.

The term "Cas9 protein" refers to Cas protein involved in a CRISPR/Cas system classified as Type II. Cas9 protein forms a complex with guide RNA and exhibits activity to cleave DNA in a target region in collaboration with the guide RNA. Cas9 protein may be any of wild-type Cas9 protein, its homologs (paralogs, orthologs, or the like), or variants thereof, as long as it has the activity to form a complex with guide RNA. Wild-type Cas9 protein has an RuvC domain and an HNH domain as nuclease domains. Cas9 protein also includes Cas9 protein having no nuclease activity.

The species from which Cas9 protein is derived is not particularly limited, but examples thereof include bacteria belonging to the genera Streptococcus, *Staphylococcus, Neisseria,* and *Treponema.* Cas9 protein is preferably derived from S. *pyogenes, S. thermophilus, S. aureus, N. meningitidis,* or *T. denticola,* and more preferably derived from S. *pyogenes.* In Cas9 protein, a sequence adjacent to the 5' side of a PAM sequence serves as a target sequence.

The term "Cas12a protein" refers to Cas protein involved in a CRISPR/Cas system classified as Type V. Cas12a protein forms a complex with guide RNA and exhibits activity to cleave DNA in a target region in collaboration with the guide RNA. Cas12a protein may be any of wild-type Cas12a protein, its homologs (paralogs, orthologs, or the like), or variants thereof, as long as it has the activity to form a complex with guide RNA. Wild-type Cas12a protein has an RuvC domain as a nuclease domain. Cas12a protein also includes Cas12a protein having no nuclease activity.

Examples of species from which Cas12a protein is derived include bacteria belonging to the genera *Acidaminococcus, Prevotella, Francisella,* and *Lachnospiraceae.* Cas12a protein is preferably derived from *Acidaminococcus sp. BV3L6.* In Cas12a protein, a sequence adjacent to the 3' side of a PAM sequence serves as a target sequence.

Amino acid sequences of various Cas proteins and information on their coding sequences can be obtained from various databases such as GenBank and UniProt. For example, as the amino acid sequence of the Cas9 protein from *S. pyogenes,* one registered in UniProt under the accession number Q99ZW2, and the like can be used. Expression vectors for various Cas proteins are commercially available, and thus commercially available ones can also be used.

The terms "guide RNA" and "gRNA" are used interchangeably and refer to RNA that forms a complex with an RNA-guided nuclease and is capable of guiding the RNA-guided nuclease to a target region. Examples of guide RNAs include RNAs that form a complex with Cas protein and are capable of guiding the Cas protein to a target region. For example, guide RNA of a Type II CRISPR/Cas system includes CRISPR RNA (crRNA) and trans-activating CRISPR RNA (tracrRNA). The crRNA is involved in binding to a target region of a genome, and the tracrRNA is involved in binding to Cas protein. In some embodiments, crRNA includes a spacer sequence and a repeat sequence, in which the spacer sequence binds to a complementary strand of a target sequence in the target region. In crRNA of a Type II CRISPR/Cas system, for example, a repeat sequence is located on the 3' side of a spacer sequence. In some embodiments, tracrRNA includes an anti-repeat sequence and a 3' tail sequence. The anti-repeat sequence has a sequence complementary to the repeat sequence of the crRNA, forming base pairs with the repeat sequence, and the 3' tail sequence typically forms three stem-loops.

Guide RNA of a Type II CRISPR/Cas system may be single-stranded guide RNA (sgRNA) in which the 5' end of tracrRNA is linked to the 3' end of crRNA, or it may consist of crRNA and tracrRNA as separate RNA molecules that form base pairs through a repeat sequence and an anti-repeat sequence. In some embodiments, guide RNA is sgRNA.

For example, guide RNA of a Type V CRISPR/Cas system (for example, a CRISPR/Cas12a system) includes crRNA but does not include tracrRNA. In crRNA of a Type V CRISPR/Cas system, for example, a repeat sequence is located on the 5' side of a spacer sequence.

The repeat sequence of crRNA and the sequence of tracrRNA can be appropriately selected depending on the type of Cas protein and may be derived from the same bacterial species as the Cas protein.

For example, when using Cas9 protein derived from S. *pyogenes,* the length of sgRNA can be about 50 to 220 nucleotides (nt), preferably about 60 to 180 nt, and more preferably about 80 to 120 nt. The length of crRNA, including a spacer sequence, can be about 25 to 70 nt, preferably about 25 to 50 nt. The length of tracrRNA can be about 10 to 130 nt, preferably about 30 to 80 nt.

The repeat sequence of a crRNA may be the same as that in the bacterial species from which the Cas protein is derived or may have a portion of the 3' end deleted. tracrRNA may have the same sequence as mature tracrRNA in the bacterial species from which the Cas protein is derived or may be a terminally truncated form in which the 5' end and/or 3' end of the mature tracrRNA is truncated. For example, tracrRNA may be a terminally truncated form in which about 1 to 40 nucleotide residues are removed from the 3' end of mature tracrRNA. In addition, tracrRNA may be a terminally truncated form in which about 1 to 80 nucleotide residues are removed from the 5' end of mature tracrRNA. In addition, tracrRNA may be a terminally truncated form in which, for example, about 1 to 20 nucleotide residues are removed from the 5' end and about 1 to 40 nucleotide residues are removed from the 3' end.

The sequences of a crRNA repeat sequence and tracrRNA for designing sgRNA have been variously proposed, and a person skilled in the art can design sgRNA based on known techniques (for example, Jinek et al. (2012) Science, 337, 816-21; Mali et al. (2013) Science, 339: 6121, 823-6; Cong et al. (2013) Science, 339: 6121, 819-23; Hwang et al. (2013) Nat. Biotechnol., 31: 3, 227-9; Jinek et al. (2013) eLife, 2, e00471).

The term "target sequence" refers to a nucleotide sequence in a genome that is subject to cleavage by an RNA-guided nuclease. In some embodiments, a target sequence refers to a nucleotide sequence in a genome that is subject to cleavage by Cas protein. A target sequence is a complementary sequence to a nucleotide sequence in the genome to which a spacer sequence of guide RNA binds. When Cas9 protein is used as Cas protein, a target sequence is a sequence adjacent to the 5' side of a protospacer adjacent motif (PAM). Typically, a sequence of 15 to 50 nucleotide residues (for example, 17 to 50 nucleotide residues, preferably 17 to 40 nucleotide residues, more preferably 17 to 30 nucleotide residues, and still more preferably 20 nucleotide residues) immediately adjacent to the 5' side of a PAM is selected as a target sequence.

When Cas12a protein is used as Cas protein, a target sequence is a sequence adjacent to the 3' side of a PAM. Typically, a sequence of 17 to 30 bases (preferably 17 to 25 bases, more preferably 19 to 22 bases, and still more preferably 20 bases) immediately adjacent to the 3' side of a PAM is selected as a target sequence.

For the design of a target sequence, known design tools such as CRISPR DESIGN (crispr.mit.edu/) can be utilized.

The term "target region" refers to a genomic region that includes a target sequence and its complementary sequence.

The terms "protospacer adjacent motif" and "PAM" are used interchangeably and refer to a sequence recognized by Cas protein during DNA cleavage by the Cas protein. The sequence and position of a PAM vary depending on the type of Cas protein. For example, in the case of Cas9 protein, a PAM is located immediately adjacent to the 3' side of a target sequence. The sequence of a PAM corresponding to Cas9 protein varies depending on the bacterial species from which the Cas9 protein is derived. For example, a PAM corresponding to the Cas9 protein from S. *pyogenes* is "NGG." A PAM corresponding to the Cas9 protein from S. *thermophilus* is "NNAGAA." A PAM corresponding to the Cas9 protein from S. *aureus* is "NNGRRT" or "NNGRR(N)." A PAM corresponding to the Cas9 protein from *N. meningitidis* is "NNNNGATT." "NAAAAC" corresponding to the Cas9 protein from *T. denticola ("R"* represents A or G; "N" represents A, T, G, or C).

In the case of Cas12a protein, a PAM is located immediately adjacent to the 5' side of a target sequence. The sequence of a PAM corresponding to Cas12a protein varies depending on the bacterial species from which the Cas12a protein is derived. For example, a PAM corresponding to the Cas12a protein from *Acidaminococcus sp. BV3L6* is "TTTV" ("V" represents A, C, or G).

The terms "spacer sequence" and "guide sequence" are used interchangeably and refer to a sequence included in guide RNA that is capable of binding to a complementary sequence of a target sequence. Typically, a spacer sequence is identical to a target sequence (provided that T in the target sequence is U in the spacer sequence). In some embodiments, a spacer sequence may include mismatches of one or multiple nucleotide residues with respect to a target sequence. When mismatches of multiple nucleotide residues are present, the mismatched nucleotide residues may be located at adjacent positions or may be located distant from each other. Examples of the length of a spacer sequence include 15 to 50 nucleotide residues, and may be 17 to 50 nucleotide residues, 17 to 40 nucleotide residues, or 17 to 30 nucleotide residues. In some embodiments, a spacer sequence is 20 nucleotide residues.

In guide RNA of a Type II CRISPR/Cas system, a spacer sequence is located on the 5' side of crRNA. In guide RNA of a Type V CRISPR/Cas system, a spacer sequence is located on the 3' side of crRNA.

The term "Cas protein having no nuclease activity" refers to Cas protein with inactivated nuclease activity. Examples of Cas proteins having no nuclease activity include Cas protein having no nuclease activity but having nickase activity (hereinafter also referred to as "nCas") and Cas protein having neither nuclease activity nor nickase activity (hereinafter also referred to as "dCas"). dCas can be obtained, for example, by mutating one or more amino acid residues of wild-type Cas protein having nuclease activity. Examples of dCas proteins include, but are not limited to, dCas9 and dCas12a. Examples of the number of amino acid substitutions include about 1 to 10, about 1 to 5, about 1 to 3, and 2. For example, when Cas protein is Cas9 protein, Cas9 protein having no nuclease activity (dCas9) may include mutations D10A, E762A, and/or D986A in the RuvC domain, and mutations H840A, N854A, and/or N863A in the HNH domain. Specific examples of dCas9 proteins include one having two amino acid substitutions of D10A and H840A.

The term "transcription regulation-related domain" refers to a domain that regulates transcription. The transcription regulation-related domain may be a transcription activation-related domain or a transcription suppression-related domain.

The transcription activation-related domain is a domain involved in transcription activation. The transcription activation-related domain may be a domain of a transcription activation factor that binds to a transcription regulation region of a target gene to activate transcription of the target gene, or a transcription activation factor recruiter domain that recruits a transcription activation factor. Examples of transcription activation-related domains include transcription activation-related domains used in CRISPR activation (CRISPRa). Examples of transcription activation-related domains include, but are not limited to, at least one selected from the group consisting of VP64, p65, Rta, and SunTag (GCN4 epitope).

The transcription suppression-related domain is a domain involved in transcription suppression. The transcription suppression-related domain may be a domain of a transcription suppression factor that binds to a transcription regulation region of a target gene to suppress transcription of the target gene, or a transcription suppression factor recruiter domain that recruits a transcription suppression factor. Examples of transcription activation-related domains include transcription suppression-related domains used in CRISPR interference (CRISPRi). Examples of transcription suppression-related domains include, but are not limited to, at least one selected from the group consisting of KRAB (the KRAB domain of Kox1) and SALL1-SDS3.

The term "CRISPR activation" (CRISPRa) refers to a system that activates transcription of a target gene using a fusion protein of dCas and a transcription activation-related domain. Examples of CRISPRa include known CRISPRa systems using VP64 as a transcription activation-related domain, those using VP64, p65, and Rta, and those using SunTag. In a CRISPRa SAM system, VP64 is used as a transcription activation-related domain, and modified guide RNA containing an MS2 RNA aptamer is used in combination with an MS2-p65-HS1 activation domain. In the CRISPRa SunTag system, GCN4 is used as a transcription activation-related domain, and a fusion protein of scFv, which specifically binds to the GCN4 epitope, and VP64 is used in combination.

The term "CRISPR interference" (CRISPRi) refers to a system that suppresses transcription of a target gene using a fusion protein of dCas and a transcription suppression-related domain. Examples of CRISPRi include known CRISPRi systems such as ones using KRAB as a transcription suppression-related domain and ones using SALL1-SDS3.

In CRISPRa and CRISPRi, guide RNA is used that targets a transcription regulation region of a target gene subject to transcription activation or transcription suppression. In some embodiments, the guide RNA of CRISPRa and CRISPRi targets a nucleotide sequence of 17 to 30 nt included in the transcription regulation region of a target gene as a target sequence. Examples of transcription regulation regions include a promoter region and a region surrounding a transcription start site (+1). Examples of the region surrounding the transcription start site include a region of 0 to 300 bp upstream and downstream of the transcription start site (-300 to +300). If transcription regulation by a transcription regulation-related domain is possible, the target sequence may be in a region further upstream than -300. Alternatively, if transcription regulation by a transcription regulation-related domain is possible, the target sequence may be in a region further downstream than +300.

The term "base editor" refers to a protein having the activity of converting a base of a nucleotide residue in a nucleic acid to another base, or a protein complex having such activity. Examples of base editors include those that perform sequence-specific base substitution using an RNA-guided nuclease with inactivated nuclease activity. Examples of base editors include proteins or protein complexes capable of performing sequence-specific base substitution using a CRISPR/Cas system. A base editor can form a complex with guide RNA and can be guided by the guide RNA to a target sequence to substitute a specific base within or near the target sequence with another base. Various base editors have been developed and are commercially available. These known base editors can be used as base editors without particular limitation. Examples of base editors include adenine base editors (which convert adenine to guanine or inosine; ABE, ABE8e, and the like), cytosine base editors (which convert cytosine to thymine; Target-AID, BE4max, and the like), and adenine transversion editors (which convert adenine to cytosine; alkyladenine DNA glycosylase/nCaas9/deaminase TadA-8e).

As a base editor, a construct is known in which a deaminase is linked to a Cas protein having no nuclease activity, either via a linker or without a linker. Examples of Cas proteins having no nuclease activity used include nCas and dCas. Examples of deaminases used include adenosine deaminase and cytidine deaminase.

The term "base editing" refers to the conversion of a base of a nucleotide residue in a nucleic acid to another base by a base editor.

The term "activity window" refers to a range in a nucleic acid subject to base editing where base editing by a base editor can occur.

The term "mismatch" refers to a situation in which a spacer sequence includes nucleotide residues that differ from those in a target sequence, or to such differing nucleotide residues. For example, the phrase "a spacer sequence including one mismatch" means that the spacer sequence differs from the target sequence by one nucleotide residue.

The term "indel" refers to an insertion and/or deletion. The term "biallelic indel" refers to a state in which an indel occurs in the target region of both alleles due to genome editing. The term "uniallelic indel" refers to a state in which an indel occurs only in the target region of one allele due to genome editing. The term "frameshift indel" refers to an indel that causes a frameshift. The term "inframe indel" refers to an indel that does not cause a frameshift.

The term "AIMS" refers to Allele-specific Indel Monitor System, a technology capable of detecting indels in an allele-specific manner. The term "AIMS cells" refers to cells constructed for performing AIMS, which are cells capable of detecting indels in an allele-specific manner.

The term "genome editing pattern" refers to the state of genome editing induced in each allele of the target region of a cell subjected to genome editing. The genome editing pattern refers to a state in which genome editing is induced in both alleles or in only one allele.

The term "functionally linked" as used with respect to a polynucleotide means that a first nucleotide sequence is arranged sufficiently close to a second nucleotide sequence to affect the second nucleotide sequence or a region under the control of the second nucleotide sequence. For example, a polynucleotide being functionally linked to a promoter means that the polynucleotide is linked so as to be expressed under the control of the promoter.

The term "expressible state" refers to a state in which a polynucleotide can be transcribed within a cell into which the polynucleotide has been introduced or in an in vitro expression system.

The term "expression vector" refers to a vector containing a target polynucleotide, equipped with a system that enables the target polynucleotide to be in an expressible state within a cell into which the vector is introduced. For example, the term "expression vector for a Cas protein" refers to a vector capable of expressing a Cas protein within a cell into which the vector is introduced or in an in vitro expression system. For example, the term "expression vector for guide RNA" refers to a vector capable of expressing guide RNA within a cell into which the vector is introduced or in an in vitro expression system.

The term "silent mutation" refers to a genetic mutation that does not change the amino acid sequence of the encoded protein.

The sequence identity between nucleotide sequences or between amino acid sequences can be determined, for example, as follows. Two nucleotide sequences or amino acid sequences are aligned by inserting gaps at positions corresponding to insertions and deletions so that the corresponding nucleotide residues or amino acid residues match as much as possible. The sequence identity is determined as the proportion of matching nucleotide residues or matching amino acid residues relative to the entire nucleotide sequence or amino acid sequence, excluding gaps in the obtained alignment. The sequence identity between nucleotide sequences or between amino acid sequences can be determined using various homology search software known in the technical field. For example, the sequence identity value of nucleotide sequences can be obtained by calculation based on an alignment obtained using the known homology search software BLASTN. The sequence identity value of amino acid sequences can be obtained by calculation based on an alignment obtained using the known homology search software BLASTP.

### [Method for controlling transcription of RNA]

A first aspect of the present disclosure is a method for controlling transcription of RNA. The method for controlling transcription of RNA includes a step of transcribing RNA from an expression vector for the RNA. The RNA is RNA to which one or more nucleotide residues are added at the 5' end.

### <RNA>

Examples of RNA include RNA functionally linked to an RNA polymerase III (pol III) promoter and capable of being transcribed by pol III. Examples of such RNA include functional RNA such as guide RNA, RNA having an RNA interference effect (siRNA, shRNA, or the like). Examples of pol III promoters include a mouse U6-snRNA promoter, a human U6-snRNA promoter, a human H1-RNase P RNA promoter, and a human valine-tRNA promoter. The RNA is preferably guide RNA. In the embodiments described below, the RNA is preferably guide RNA. When the RNA is guide RNA of a CRISPR/Cas system, nucleotide residues are added to the 5' end of crRNA. The 5' end of the RNA refers to the 5' end of an RNA molecule capable of expressing its function as functional RNA.

By adjusting the number and type of nucleotide residues to be added to the 5' end of RNA, the transcription efficiency of RNA can be controlled in an in vitro transcription system or in cells into which an expression vector has been introduced. The cells into which an expression vector is introduced may be cells in a living body. The introduction of an expression vector may be performed in vitro, ex vivo, or in vivo.

In another aspect, the present disclosure provides a method for designing an expression vector for RNA. The method for designing DNA includes: designing an expression vector for RNA in which transcription efficiency of the RNA is controlled by adjusting one or more selected from the group consisting of the number of nucleotide residues to be added to the 5' end of an RNA coding sequence and the type of the nucleotide residues. Examples of the RNA include those described above, with guide RNA being preferred. For example, by preparing an RNA coding sequence without an added nucleotide residue coding sequence at the 5' end, or with varying numbers and/or types of added nucleotide residues, and conducting an RNA expression test using these, an expression vector that achieves a desired expression level can be designed.

As the RNA, one to which one or more nucleotide residues are added at the 5' end is used. The number of nucleotide residues to be added (hereinafter also referred to as "added nucleotide residues") includes, for example, a range of 1 to 50. The number of added nucleotide residues can be, for example, 3 or more, 5 or more, 10 or more, 15 or more, 20 or more, or 25 or more. The upper limit of the number of added nucleotide residues includes, for example, 50 or less, preferably 40 or less, more preferably 35 or less, and still more preferably 30 or less. The upper limit and lower limit values can be arbitrarily combined. Preferred ranges for the number of added nucleotide residues include, for example, 3 to 50, 5 to 40, 5 to 35, and 5 to 30.

In the method for controlling transcription of RNA of the present embodiment, the transcription of RNA can be controlled by adjusting the number of added nucleotide residues. For example, the greater the number of added nucleotide residues, the more the transcription of RNA is suppressed. For example, by increasing or decreasing the number of added nucleotide residues within a range of 0 to 50, 0 to 30, or 0 to 25, the transcription efficiency of RNA can be precisely adjusted. When a greater transcription suppression effect of RNA is desired, the number of added nucleotide residues can be, for example, 5 or more, 10 or more, 15 or more, 20 or more, or 25 or more.

The type of added nucleotide residues may be any of adenine nucleotide residues (A), uracil nucleotide residues (U), guanine nucleotide residues (G), and cytosine nucleotide residues (C), or a combination of two or more thereof. When the number of added nucleotide residues is two or more, the added nucleotide residues may include a region where the same type of nucleotide residues are consecutive (hereinafter also referred to as a "consecutive region"). The type of consecutive nucleotide residues may be any of adenine nucleotide residues (A), uracil nucleotide residues (U), guanine nucleotide residues (G), and cytosine nucleotide residues (C). The number of consecutive nucleotide residues has the total number of added nucleotide residues as an upper limit value and includes, for example, 2 or more, 3 or more, 4 or more, 5 or more, 10 or more, 15 or more, 20 or more, and 25 or more. The percentage of the consecutive region in the total added nucleotide residues includes, for example, 10% to 100%, 20% to 100%, 30% to 100%, 40% to 100%, 50% to 100%, 60% to 100%, 70% to 100%, 80% to 100%, and 90% to 100%.

Hereinafter, a region where the same type of nucleotide residues are consecutive may be denoted as [nN]. n represents the number of consecutive nucleotide residues, and N represents the type of consecutive nucleotide residues. For example, [10C] indicates that 10 cytosine nucleotide residues are consecutive. When denoted as [nC], it indicates that n cytosine nucleotide residues are consecutive. n is any integer of 2 or more. [nN] may include 1 to 2 nucleotide residues of a different type.

The transcription suppression effect of RNA tends to be stronger with [nC] and [nU] (or [nT]), followed by [nG], and weaker with [nA]. Therefore, by appropriately selecting or combining [nC], [nU] (or [nT]), [nG], and [nA], the desired RNA expression level can be adjusted.

The added nucleotide residues may not include a consecutive region. The added nucleotide residues may, for example, be such that the RNA forms a hairpin structure.

When using an expression vector encoding RNA, it is preferable that the added polynucleotide residues do not include a complementary sequence to a terminator sequence that stops transcription from the promoter used. For example, when using a U6 promoter, transcription stops when five thymines are consecutive, and thus it is preferable that the added nucleotide residues do not include five or more consecutive uracil nucleotide residues ([nU]: n ≥ 5).

### <Expression vector for RNA>

An RNA expression vector (for example, an expression vector for guide RNA) is an expression vector capable of expressing RNA (for example, guide RNA) having added nucleotide residues at the 5' end as described above when introduced into a cell. The RNA expression vector (for example, an expression vector for guide RNA) preferably includes a sequence encoding RNA (for example, guide RNA) (an RNA coding sequence (for example, a guide RNA coding sequence)) and a promoter that controls the expression of the RNA coding sequence. In the RNA expression vector (for example, an expression vector for guide RNA), the RNA coding sequence (for example, a guide RNA coding sequence) is functionally linked to the promoter.

The promoter is not particularly limited as long as it has a function of expressing RNA (for example, guide RNA). Examples of the promoter include an RNA polymerase II (pol II) promoter and an RNA polymerase III (pol III) promoter. Using a pol III promoter allows for more accurate transcription of relatively short RNA. Examples of pol III promoters include a mouse U6-snRNA promoter, a human U6-snRNA promoter, a human H1-RNase P RNA promoter, and a human valine-tRNA promoter. When using a U6 promoter, the 5' end of RNA (for example, guide RNA) is preferably a guanine nucleotide residue (G) for transcription start. Therefore, the 5' end of added nucleotide residues of RNA (for example, guide RNA) is preferably a guanine nucleotide residue.

The RNA expression vector (for example, an expression vector for guide RNA) may have other components in addition to an RNA coding sequence (for example, a guide RNA coding sequence) and a promoter. Examples of other components include a terminator, an enhancer, a marker gene, a replication origin, and a gene encoding a protein that binds to the replication origin to control replication. The terminator is linked to the 3' side of an RNA coding sequence (for example, a guide RNA coding sequence). As the terminator, one commonly used as a terminator for RNA (for example, guide RNA) can be used. The term "marker gene" refers to a gene that enables the selection or screening of cells by introducing the marker gene into the cells. Specific examples of marker genes include drug resistance genes, fluorescent protein genes, luminescent enzyme genes, and chromogenic enzyme genes. These may be used alone or in combination of two or more thereof. Specific examples of the drug resistance genes include a puromycin resistance gene, a geneticin resistance gene, a neomycin resistance gene, a tetracycline resistance gene, a kanamycin resistance gene, a zeocin resistance gene, a hygromycin resistance gene, and a chloramphenicol resistance gene. Specific examples of the fluorescent protein genes include a green fluorescent protein (GFP) gene, a yellow fluorescent protein (YFP) gene, and a red fluorescent protein (RFP) gene. Specific examples of the luminescent enzyme genes include a luciferase gene. Specific examples of the chromogenic enzyme genes include a β-galactosidase gene, a β-glucuronidase gene, and an alkaline phosphatase gene.

The type of expression vector is not particularly limited, and known expression vectors can be used. Examples of expression vectors include plasmid vectors and viral vectors.

The plasmid vector is not particularly limited as long as it is a plasmid vector capable of being expressed in a cell to be subjected to genome editing. For example, in the case of animal cells, a plasmid vector commonly used for animal cell expression can be used. Examples of plasmid vectors for animal cell expression include pX459, pA1-11, pXT1, pRc/CMV, pRc/RSV, and pcDNAI/Neo, but are not limited thereto.

Examples of viral vectors include retrovirus (including lentivirus) vectors, adenovirus vectors, adeno-associated virus vectors, Sendai virus vectors, herpesvirus vectors, vaccinia virus vectors, poxvirus vectors, poliovirus vectors, sindbis virus vectors, rhabdovirus vectors, paramyxovirus vectors, and orthomyxovirus vectors.

Among these, a plasmid vector is preferred as the expression vector.

### <RNA Transcription step>

The expression of RNA (for example, guide RNA) from an expression vector for RNA (for example, guide RNA) can be performed by known methods. The expression of RNA (for example, guide RNA) may be performed in an extracellular transcription system or within a cell. When expressing RNA (for example, guide RNA) in an extracellular transcription system, an RNA polymerase (for example, pol III) may be applied to the expression vector for RNA (for example, guide RNA). When expressing RNA (for example, guide RNA) within a cell, the expression vector for RNA (for example, guide RNA) may be introduced into a desired cell. The cell may be an in vitro cell or an in vivo cell. This step may be performed in vitro, ex vivo, or in vivo. When the transcription of RNA is performed within a cell, the method for controlling transcription of RNA includes a step of introducing an expression vector for RNA into the cell.

When RNA is guide RNA, the method for controlling transcription of guide RNA may include a step of introducing, into a cell, (A) an expression vector for guide RNA to which one or more nucleotide residues are added at the 5' end, and (B) at least one selected from the group consisting of an RNA-guided nuclease, mRNA of the RNA-guided nuclease, and an expression vector for the RNA-guided nuclease. The cell may be an in vivo cell. This step may be performed in vitro, ex vivo, or in vivo.

### <RNA-Guided nuclease>

Examples of RNA-guided nucleases include Cas protein. Cas protein is not particularly limited as long as it is used in a CRISPR/Cas system. Cas protein may be from any of Type I to Type VI CRISPR/Cas systems. Cas protein is not limited to one having endonuclease activity and may be nCas or dCas. In some embodiments, Cas protein is Cas protein of a Type II or Type V CRISPR/Cas system. In some embodiments, Cas protein is Cas9 protein or Cas12a protein.

Cas protein may be a variant of wild-type Cas protein as long as it is capable of forming a complex with guide RNA. Examples of variants of Cas protein include the following proteins (b1) or (b2).

(b1) A protein consisting of an amino acid sequence having, for example, 85% or greater, preferably 90% or greater, more preferably 95% or greater, and still more preferably 98% or greater sequence identity with the amino acid sequence of wild-type Cas protein, and having the ability to form a complex with guide RNA.

(b2) A protein consisting of an amino acid sequence in which one or more (for example, 2 to 100, preferably 2 to 50, more preferably 2 to 20, still more preferably 2 to 10, still more preferably 2 to 5, and particularly preferably 2) amino acids are substituted, deleted, added, or inserted relative to the amino acid sequence of wild-type Cas protein, and having the ability to form a complex with guide RNA.

### <Expression vector for RNA-Guided Nuclease>

An expression vector for an RNA-guided nuclease is an expression vector capable of expressing an RNA-guided nuclease when introduced into a cell. The expression vector for an RNA-guided nuclease preferably includes a coding sequence for the RNA-guided nuclease (RNA-guided nuclease coding sequence) and a promoter that controls the expression of the RNA-guided nuclease coding sequence. In the expression vector for an RNA-guided nuclease, the RNA-guided nuclease coding sequence is functionally linked to the promoter. The RNA-guided nuclease is preferably Cas protein.

The promoter is not particularly limited as long as it has the function of expressing an RNA-guided nuclease (for example, Cas protein). Examples of the promoter include a pol II promoter. Examples of the pol II promoter include a CMV promoter, an EF1 promoter, an SV40 promoter, an MSCV promoter, an hTERT promoter, a β-actin promoter, a CAG promoter, and a CBh promoter.

The expression vector for an RNA-guided nuclease may have other components in addition to the RNA-guided nuclease coding sequence and the promoter. Examples of other components include a terminator, an enhancer, a poly(A) addition signal, a marker gene, a replication origin, and a gene encoding a protein that binds to the replication origin to control replication. The terminator is linked to the 3' side of the RNA-guided nuclease coding sequence. As the terminator, one commonly used as a terminator for an RNA-guided nuclease (for example, Cas protein) can be used. Examples of the marker gene include the same ones described above.

The type of expression vector is not particularly limited, and known expression vectors can be used. Examples of expression vectors include plasmid vectors and viral vectors. Examples of these vectors include the same ones described above.

Among these, a plasmid vector is preferred as the expression vector.

The RNA-guided nuclease coding sequence (for example, Cas protein coding sequence) included in the expression vector may be codon-optimized according to the species of a target cell. Codon optimization refers to replacing at least one codon of an original nucleotide sequence with a codon more frequently used in a target species while maintaining an original amino acid sequence. Codon usage frequency tables are readily available, for example, from the "Codon Usage Database" (www.kazusa.or.jp/codon/) provided by the Kazusa DNA Research Institute. Codons can be optimized using these known codon usage frequency tables. Computer algorithms for codon optimization of specific sequences for expression in specific animal species are available, for example, from Gene Forge (Aptagen, LLC.; Jacobus, PA).

### <Expression vector for guide RNA and RNA-guided nuclease>

When using an expression vector for an RNA-guided nuclease (for example, Cas protein), an expression vector for guide RNA and an expression vector for an RNA-guided nuclease may be the same expression vector. In some embodiments, an expression vector is an expression vector capable of expressing guide RNA and an RNA-guided nuclease (hereinafter also referred to as a "guide RNA/RNA-guided nuclease expression vector"). The guide RNA/RNA-guided nuclease expression vector includes a guide RNA coding sequence and an RNA-guided nuclease coding sequence, each in an expressible state. The guide RNA/RNA-guided nuclease expression vector includes a guide RNA coding sequence functionally linked to a promoter for guide RNA and an RNA-guided nuclease coding sequence functionally linked to a promoter for an RNA-guided nuclease. A terminator for guide RNA may be linked to the 3' side of the guide RNA coding sequence. A terminator for an RNA-guided nuclease coding sequence may be linked to the 3' side of the RNA-guided nuclease coding sequence.

The guide RNA/RNA-guided nuclease expression vector may further include other components. Examples of other components include the same ones described above.

### <Introduction step>

The method of the present embodiment may include a step of introducing into a cell: (A) the expression vector for guide RNA; and (B) at least one selected from the group consisting of an RNA-guided nuclease (for example, Cas protein), mRNA of the RNA-guided nuclease, and an expression vector for an RNA-guided nuclease.

The cell into which the above-described (A) and (B) are introduced is not particularly limited. An organism from which the cell is derived is not particularly limited and includes, for example, animals (such as mammalian animals such as humans, monkeys, mice, rats, dogs, cats, rabbits, cows, horses, pigs, goats, and sheep; avian animals such as chickens; reptilian animals such as snakes and lizards; amphibian animals such as African clawed frogs; fishes such as zebrafish, medaka, and pufferfish; chordates such as ascidians; and arthropods such as fruit flies and silkworms); plants such as *Arabidopsis,* rice, wheat, and tobacco; fungi such as yeast and Neurospora; and bacteria such as *Escherichia coli, Bacillus subtilis,* and cyanobacteria.

The type of cell is not particularly limited and includes, for example, cells derived from various tissues or having various properties, such as blood cells, hematopoietic stem/progenitor cells, gametes (sperm, oocytes), fertilized eggs, fibroblasts, epithelial cells, vascular endothelial cells, nerve cells, hepatocytes, keratinocytes, muscle cells, epidermal cells, endocrine cells, tissue stem cells, iPS cells, ES cells, and cancer cells. In addition, cells having various hereditary diseases such as sickle cell anemia, Huntington's disease, Duchenne muscular dystrophy, and fibrodysplasia ossificans progressiva (FOP) are included.

The method for introducing the above-described (A) and (B) can be appropriately selected depending on a target cell and the type of material (for example, whether it is a nucleic acid or a protein).

Examples of methods for introducing an expression vector into a cell include a lipofection method, a microinjection method, a DEAE-dextran method, a gene gun method, an electroporation method, and a calcium phosphate method. When the expression vector is a viral vector, a method of infecting a cell with the viral vector (for example, a polybrene method) can be used.

The method for introducing RNA into a cell is not particularly limited, and known methods can be appropriately selected and used. For example, for RNA introduction, commercially available RNA transfection reagents such as Lipofectamine (registered trademark) MessengerMAX (manufactured by Life Technologies Corporation) can be used.

The method for introducing a protein into a cell is not particularly limited, and known methods can be appropriately selected and used. Examples of such a method include a method using a protein transduction reagent, a method using a protein transduction domain (PTD) fusion protein, and a microinjection method.

The above-described (A) and (B) may be introduced into a cell simultaneously, sequentially, or separately with a certain interval. In some embodiments, the above-described (A) and (B) are introduced into a cell simultaneously.

### <Other steps>

The method of the present embodiment may include other steps in addition to the above-described introduction step. Examples of other steps include a step of introducing a donor vector into a cell, a step of culturing a cell, and a step of measuring the expression level of guide RNA.

### <<Donor vector introduction step>>

When RNA is guide RNA, the method for controlling transcription of guide RNA may include a step of introducing a donor vector into a cell. The donor vector includes a nucleotide sequence adjacent to a target region as a homology arm. The donor vector can include a desired nucleotide sequence (hereinafter also referred to as a "knock-in sequence") between a 5' homology arm and a 3' homology arm. The knock-in sequence is not particularly limited and can be any sequence. The knock-in sequence may be, for example, a sequence for gene knockout, a sequence for introducing one or more mutations, or any gene sequence. When the knock-in sequence is any gene sequence, it is preferable to set a target sequence within a safe harbor region.

The donor vector may be a circular DNA vector (for example, a plasmid vector) or a linear DNA vector. The donor vector may include other components in addition to the homology arm and the knock-in sequence. Examples of other components include same ones as the expression vectors described above.

The method for introducing a donor vector is not particularly limited and can be appropriately selected depending on a target cell. Examples of methods for introducing a donor vector into a cell include a lipofection method, a microinjection method, a DEAE-dextran method, a gene gun method, an electroporation method, and a calcium phosphate method.

A donor vector may be introduced into a cell simultaneously with the above-described (A) and (B), sequentially, or with a certain interval after the introduction of (A) and (B). In some embodiments, a donor vector is introduced into a cell simultaneously with the above-described (A) and (B).

### <<Culture step>>

When transcription of RNA is performed within a cell, the method for controlling transcription of RNA may include a step of culturing a cell after introducing an expression vector for RNA into the cell. When the method for controlling transcription of RNA includes a step of introducing the above-described (A) and (B) into a cell, the method for controlling transcription of RNA may include a step of culturing a cell after introducing the above-described (A) and (B), and, if necessary, a donor vector into the cell. The cell culture can be performed under appropriate culture conditions depending on the type of cell. When an expression vector for RNA, or any one or more of the above-described (A), (B), and the donor vector is a vector containing a drug resistance marker, the culture may be performed in the presence of the drug. By performing the culture in the presence of the drug, cells into which a vector has been introduced can be efficiently selected. In addition, cell cloning may be performed by dilution of the cell culture medium, plating, or the like.

### <<RNA Expression level measurement step>>

The method for controlling transcription of RNA may include a step of measuring the expression level of RNA. When the method for controlling transcription of RNA includes a step of introducing the above-described (A) and (B) into a cell, the method for controlling transcription of RNA may include a step of measuring the expression level of guide RNA in the cell after introducing the above-described (A) and (B), and, if necessary, a donor vector into the cell. The expression level of RNA can be measured by known methods such as Northern blot analysis and quantitative reverse transcription PCR (RT-qPCR). The expression level of RNA is considered to reflect the transcription level of RNA. Therefore, the number and type of added nucleotide residues can be adjusted so that RNA is transcribed at a desired transcription level based on the measured expression level of RNA.

According to the method for controlling transcription of RNA of the present embodiment, the transcription level of RNA from an expression vector for RNA can be controlled by a simple method of adjusting the number and type of added nucleotide residues.

### [Method for controlling transcription of target gene]

A second aspect of the present disclosure is a method for controlling transcription of a target gene. The method for controlling transcription of a target gene includes: a step of introducing, into a cell, (A) at least one selected from the group consisting of (a1) guide RNA to which one or more nucleotide residues are added at the 5' end, and (a2) an expression vector for the guide RNA of (al); and (B) at least one selected from the group consisting of a fusion protein including an RNA-guided nuclease with inactivated nuclease activity and a transcription regulation-related domain, mRNA of the fusion protein, and an expression vector for the fusion protein. The guide RNA targets a transcription regulation region of the target gene.

### <Guide RNA>

Guide RNA the same as that described in the above [Method for controlling transcription of RNA] can be used. In the method for controlling transcription of a target gene, guide RNA targets a transcription regulation region of the target gene. The target sequence in the transcription regulation region targeted by guide RNA can be appropriately set depending on the type of the target gene. In some embodiments, the target sequence may be set, for example, within a region of -1000 to +300 relative to a transcription start point (+1) of the target gene. If transcription regulation by a transcription regulation-related domain is possible, the target sequence may be set outside the range of the region. The number and type of added nucleotides in guide RNA can be the same as those described in the above [Method for controlling transcription of RNA].

In some embodiments, the method for controlling transcription of a target gene utilizes CRISPRa or CRISPRi. The effect of CRISPRa and CRISPRi can be controlled by adjusting the number and type of added nucleotide residues in guide RNA. This enables control of the transcription level of the target gene.

For example, when utilizing CRISPRa, the greater the number of added nucleotide residues in guide RNA, the more the transcription activation by CRISPRa is suppressed. Therefore, by adjusting the number and type of added nucleotide residues, transcription activation by CRISPRa can be precisely controlled. This enables control of the transcription of a target gene.

For example, when utilizing CRISPRi, the greater the number of added nucleotide residues in guide RNA, the more the transcription suppression effect by CRISPRi is reduced. Therefore, by adjusting the number and type of added nucleotide residues, transcription suppression by CRISPRi can be precisely controlled. This enables control of the transcription of a target gene.

For example, by increasing or decreasing the number of added nucleotide residues in guide RNA within a range of 0 to 50, 0 to 30, 0 to 25, 0 to 20, 0 to 15, or 0 to 10, transcription activation by CRISPRa or transcription suppression by CRISPRi can be precisely adjusted.

### <Expression vector for guide RNA>

As an expression vector for guide RNA, the expression vector for RNA described in the above [Method for controlling transcription of RNA] in which guide RNA is used as the RNA, can be used.

### <RNA-Guided nuclease with inactivated nuclease activity>

As an RNA-guided nuclease with inactivated nuclease activity, it is preferable to use an RNA-guided nuclease that has inactivated nuclease activity and does not have nickase activity. Examples of the RNA-guided nuclease with inactivated nuclease activity include Cas protein having no nuclease activity. As Cas protein having no nuclease activity, it is preferable to use dCas. Examples of dCas include those obtained by modifying wild-type Cas protein to have neither nuclease activity nor nickase activity.

As dCas, those commonly used in CRISPRa or CRISPRi can be used without particular limitation. dCas may be derived from Cas protein of any of Type I to Type VI CRISPR/Cas systems. In some embodiments, the Cas protein from which dCas is derived is Cas protein of a Type II or Type V CRISPR/Cas system. In some embodiments, dCas may be Cas9 protein having neither nuclease activity nor nickase activity (dCas9) or Cas12a protein having neither nuclease activity nor nickase activity (dCas12a or dCpf1).

### <Transcription regulation-related domain>

As a transcription regulation-related domain, those commonly used in CRISPRa or CRISPRi can be used without particular limitation. In CRISPRa, a transcription activation-related domain is used as the transcription regulation-related domain. In CRISPRi, a transcription suppression-related domain is used as the transcription regulation-related domain.

### <Fusion protein including Cas protein having no nuclease activity and transcription regulation-related domain>

In a fusion protein, a transcription regulation-related domain may be arranged on the N-terminal side or the C-terminal side of Cas protein having no nuclease activity. A fusion protein may include other domains in addition to Cas protein having no nuclease activity and a transcription regulation-related domain. Examples of other domains include a nuclear localization signal domain.

### <Expression vector for fusion protein>

An expression vector for a fusion protein is an expression vector capable of expressing a fusion protein when introduced into a cell. The expression vector for a fusion protein preferably includes a coding sequence for the fusion protein (fusion protein coding sequence) and a promoter that controls the expression of the fusion protein coding sequence. In the expression vector for a fusion protein, the fusion protein coding sequence is functionally linked to the promoter.

The expression vector for a fusion protein can have the same configuration as the expression vector for an RNA-guided nuclease described in the above [Method for controlling transcription of RNA], except that it includes a fusion protein coding sequence instead of an RNA-guided nuclease coding sequence.

### <Expression vector for guide RNA and fusion protein>

When using an expression vector for a fusion protein, an expression vector for guide RNA and an expression vector for a fusion protein may be the same expression vector. In some embodiments, the expression vector is an expression vector capable of expressing guide RNA and a fusion protein (hereinafter also referred to as a "guide RNA/fusion protein expression vector"). A guide RNA/fusion protein expression vector includes a guide RNA coding sequence and a fusion protein coding sequence, each in an expressible state. A guide RNA/fusion protein expression vector includes a guide RNA coding sequence functionally linked to a promoter for guide RNA and a fusion protein coding sequence functionally linked to a promoter for a fusion protein. A terminator for guide RNA may be linked to the 3' side of the guide RNA coding sequence. A terminator for a fusion protein coding sequence may be linked to the 3' side of the RNA-guided nuclease coding sequence.

An expression vector for guide RNA and a fusion protein may further include other components. Examples of other components include the same ones described above.

### <Introduction step>

The method of the present embodiment includes: a step of introducing, into a cell, (A) at least one selected from the group consisting of (a1) guide RNA to which one or more nucleotide residues are added at the 5' end, and (a2) an expression vector for the guide RNA of (al); and (B) at least one selected from the group consisting of a fusion protein including an RNA-guided nuclease with inactivated nuclease activity and a transcription regulation-related domain, mRNA of the fusion protein, and an expression vector for the fusion protein.

The introduction step can be performed in the same manner as in the <Introduction step> described in the above [Method for controlling transcription of RNA]. This step may be performed in vitro, ex vivo, or in vivo.

### <Other steps>

The method of the present embodiment may include other steps in addition to the above-described introduction step. Examples of other steps include a step of culturing a cell and a step of measuring the expression level of a target gene.

The step of culturing a cell can be performed in the same manner as in the <<Culture step>> described in the above [Method for controlling transcription of RNA].

The step of measuring the expression level of a target gene can be performed by introducing the above-described (A) and (B) into a cell, followed by measuring the expression level of the target gene in the cell. The expression level of the target gene in the cell can be measured by known methods such as Northern blot analysis and quantitative reverse transcription PCR (RT-qPCR). The expression level of the target gene is considered to reflect the transcription level of the target gene. Therefore, the number and type of added nucleotide residues can be adjusted so that the target gene is transcribed at a desired transcription level based on the measured expression level of the target gene.

According to the method for controlling transcription of a target gene of the present embodiment, in a system for controlling transcription of a target gene using an RNA-guided nuclease and guide RNA, the transcription level of the target gene can be more precisely controlled by a simple method of adjusting the number and type of added nucleotide residues. The method for controlling transcription of a target gene of the present embodiment can more precisely control the transcription of a target gene, particularly in a system using CRISPRa or CRISPRi.

### [Genome editing method]

A third aspect of the present disclosure is a genome editing method. The genome editing method includes: a step of introducing, into a cell, (A) at least one selected from the group consisting of (a1) guide RNA which has a repeat sequence on the 5' side of a spacer sequence and to which one or more nucleotide residues are added at the 5' end, and (a2) an expression vector for the guide RNA of (a1); and (B) at least one selected from the group consisting of an RNA-guided nuclease, mRNA of the RNA-guided nuclease, and an expression vector for the RNA-guided nuclease.

### <Guide RNA>

Guide RNA in the genome editing method of the present embodiment is guide RNA having a repeat sequence on the 5' side of a spacer sequence. Examples of such guide RNA include guide RNA of a Type V CRISPR/Cas system. In some embodiments, the guide RNA does not include tracrRNA. In some embodiments, the spacer sequence includes a nucleotide sequence capable of binding to a complementary sequence of a target sequence adjacent to the 3' side of a PAM. In some embodiments, the guide RNA is guide RNA for Cas12a.

The guide RNA has added nucleotide residues at the 5' end. The added nucleotide residues are the same as those described in the above [Method for controlling transcription of RNA]. The number and type of added nucleotide residues in guide RNA can be the same as those exemplified in the above [Method for controlling transcription of RNA].

In the genome editing method of the present embodiment, the genome editing pattern can be controlled by adjusting the number and type of added nucleotide residues. For example, as the number of added nucleotide residues increases, the proportion of cells in which only one allele is genome-edited can be increased. For example, by increasing or decreasing the number of added nucleotide residues within a range of 3 to 50, 5 to 50, 5 to 40, or 5 to 30, the genome editing pattern can be precisely adjusted.

### <Expression vector for guide RNA>

An expression vector for guide RNA the same as the expression vector for RNA described in the above [Method for controlling transcription of RNA] in which guide RNA is used as RNA can be used, except that a guide RNA coding sequence is a coding sequence for the guide RNA described above.

### <RNA-Guided nuclease>

Examples of RNA-guided nucleases include Cas protein. Cas protein is not particularly limited as long as it is used in a CRISPR/Cas system. Cas protein may be from any of Type I to Type VI CRISPR/Cas systems. Cas protein is not limited to one having endonuclease activity and may have nickase activity or may be dCas. In some embodiments, Cas protein is Cas protein of a Type V CRISPR/Cas system. In some embodiments, Cas protein is Cas12a protein.

Cas protein may be a protein of (b1) or (b2) in the above-described [Method for controlling transcription of RNA]. Examples of wild-type Cas protein in (b1) or (b2) include Cas12a protein.

### <Expression vector for RNA-Guided Nuclease>

An expression vector for an RNA-guided nuclease the same as that described in the above [Method for controlling transcription of RNA] can be used. The expression vector for an RNA-guided nuclease is preferably an expression vector for Cas protein.

### <Expression vector for guide RNA and RNA-guided nuclease>

When using an expression vector for an RNA-guided nuclease, an expression vector for guide RNA and an expression vector for an RNA-guided nuclease may be the same expression vector. An expression vector for guide RNA/an RNA-guided nuclease the same as that described in the above [Method for controlling transcription of RNA] can be used, except that an RNA coding sequence is the above-described guide RNA coding sequence.

### <Introduction step>

The genome editing method of the present embodiment includes: a step of introducing, into a cell, (A) at least one selected from the group consisting of (a1) guide RNA which has a repeat sequence on the 5' side of a spacer sequence and to which one or more nucleotide residues are added at the 5' end, and (a2) an expression vector for the guide RNA of (al); and (B) at least one selected from the group consisting of an RNA-guided nuclease, mRNA of the RNA-guided nuclease, and an expression vector for the RNA-guided nuclease.

The introduction step can be performed in the same manner as in the <Introduction step> described in the above [Method for controlling transcription of RNA]. This step may be performed in vitro, ex vivo, or in vivo.

### <Other steps>

The genome editing method of the present embodiment may include other steps in addition to the above-described introduction step. Examples of other steps include a step of introducing a donor vector into a cell, a step of culturing a cell, and a step of analyzing a genome editing pattern.

A donor vector the same as that in the <<Donor vector introduction step>> described in the above [Method for controlling transcription of RNA] can be used.

The step of introducing a donor vector into a cell can be performed in the same manner as in the <<Donor vector introduction step>> described in the above [Method for controlling transcription of RNA].

The step of culturing a cell can be performed in the same manner as in the <<Culture step>> described in the above [Method for controlling transcription of RNA].

The step of analyzing a genome editing pattern can be performed by known genome editing pattern analysis methods after the above-described introduction step. Examples of genome editing pattern analysis methods include a method in which, after performing a culture step as appropriate, plating of a cell culture solution is conducted, DNA is extracted from the resulting colonies, and sequence analysis of a target region is performed.

According to the genome editing method of the present embodiment, in a genome editing method using guide RNA having a repeat sequence on the 5' side of a spacer sequence, the genome editing pattern can be precisely controlled by a simple method of adjusting the number and type of added nucleotide residues. Furthermore, according to the genome editing method of the present embodiment, an off-target suppression effect can also be expected.

### [Base editing method]

A fourth aspect of the present disclosure is a base editing method. The base editing method includes: a step of introducing, into a cell, (A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (al); and (B) at least one selected from the group consisting of a base editor, mRNA of the base editor, and an expression vector for the base editor.

### <Guide RNA>

Guide RNA in which one or more selected from the group consisting of (1) addition of nucleotide residues to the 5' end, (2) length of a spacer sequence, and (3) introduction of mismatched nucleotide residues in the spacer sequence are adjusted is used as guide RNA.

In some embodiments, a base editor utilizing a CRISPR/Cas system is used in the base editing method. By adjusting one or more of the (1) to (3) in the guide RNA, an activity window of the base editor can be controlled. In some embodiments, a target sequence of guide RNA is set to a region including nucleotide residues to be subjected to base editing by a base editor (hereinafter also referred to as "target nucleotide residues") or a region near target nucleotide residues (for example, near the 3' side). The target sequence is preferably designed so that the target nucleotide residue falls within the activity window of the base editor.

### <<(1) Addition of nucleotide residues to 5' end>>

By adding nucleotide residues to the 5' end of guide RNA, an activity window of a base editor can be controlled. The number and type of added nucleotide residues in guide RNA can be the same as those exemplified in the above [Method for controlling transcription of RNA]. When a base editor includes nCas, the added nucleotide residues in guide RNA are added to the 5' end of crRNA.

For example, as the number of added nucleotide residues increases, the activity window of a base editor tends to become narrower. When attempting to narrow the activity window of a base editor, the number of added nucleotide residues can be, for example, 3 or more, 4 or more, 5 or more, 10 or more, 15 or more, or 20 or more. The upper limit of the number of added nucleotide residues includes, for example, 50 or less, preferably 40 or less, more preferably 35 or less, and still more preferably 30 or less. The upper limit and lower limit values can be arbitrarily combined. When attempting to narrow the activity window of a base editor, the range of the number of added nucleotide residues includes, for example, 3 to 50, 5 to 40, 5 to 35, 5 to 30, 10 to 30, or 15 to 30.

The type of added nucleotide residues may be any of adenine nucleotide residues (A), uracil nucleotide residues (U), guanine nucleotide residues (G), and cytosine nucleotide residues (C), or a combination of two or more thereof. The added nucleotide residues may include a region where the same type of nucleotide residues are consecutive (consecutive region; [nN]). When attempting to narrow the activity window of a base editor, the added nucleotide residues preferably include [nN]. [nN] may be any of [nC], [nU] (or [nT]), [nG], and [nA], but [nC] is preferred. The value of n in [nN] has the total number of added nucleotide residues as an upper limit value and includes, for example, 2 or more, 3 or more, 4 or more, 5 or more, 10 or more, 15 or more, 20 or more, and 25 or more. The percentage of the consecutive region in the total added nucleotide residues includes, for example, 10% to 100%, 20% to 100%, 30% to 100%, 40% to 100%, 50% to 100%, 60% to 100%, 70% to 100%, 80% to 100%, and 90% to 100%.

The added nucleotide residues also have an effect of shifting an activity window of a base editor to the 3' side or the 5' side relative to a nucleic acid strand including a target sequence. When attempting to shift the activity window of a base editor to the 3' side relative to the nucleic acid strand including the target sequence, the number of added nucleotide residues can be, for example, 3 or more, 4 or more, 5 or more, 10 or more, 15 or more, 20 or more, or 25 or more, and can be, for example, 50 or less, 40 or less, or 30 or less. The upper limit and lower limit values can be arbitrarily combined. When attempting to shift the activity window of a base editor to the 3' side of the target sequence, the range of the number of added nucleotide residues includes, for example, 3 to 50, 5 to 40, 5 to 35, 5 to 30, 10 to 30, 15 to 30, 20 to 30, or 25 to 30. The number of added nucleotide residues is preferably 15 to 30, and more preferably 20 to 30. By adjusting the number and type of added nucleotide residues, the activity window of a base editor can also be shifted to the 5' side relative to the nucleic acid strand including the target sequence.

When attempting to shift the activity window of a base editor to the 3' side, [nN] may be added to the 5' end, and further nucleotide residues may be added to the 5' end of [nN]. The type of nucleotide residues added to the 5' end of [nN] may be any of adenine nucleotide residues (A), uracil nucleotide residues (U), guanine nucleotide residues (G), and cytosine nucleotide residues (C), or a combination of two or more thereof. The number of nucleotide residues added to the 5' end of [nN] includes, for example, 1 or more, 2 or more, or 3 or more, and 20 or less, 15 or less, 10 or less, or 5 or less. The upper and lower limit values can be arbitrarily combined. The number of nucleotide residues added to the 5' end of [nN] includes, for example, 1 to 20, 1 to 15, 1 to 10, 1 to 9, 1 to 8, 1 to 7, 1 to 6, 1 to 5, 1 to 4, 1 to 3, or 1 to 2.

Hereinafter, the nucleotide residues added to the 5' end of [nN] may be represented as [nX]. n represents the number of nucleotide residues. X represents any nucleotide residue. When n is 2 or more, the two or more X's may be the same as or different from each other. In some embodiments, the added nucleotide residues include a nucleotide sequence represented as [nX][nN].

Specific examples of [nX] include one selected from the group consisting of CUU (or CTT), AGC, and CGG. Specific examples of [nX][nN] include CUU[nN], AGC[nN], and CGG[nN]. The above [nN] may be any of [nC], [nU] (or [nT]), [nG], and [nA], but is preferably [nC]. Examples of the value of n in [nN] in [nX][nN] include 5 to 50, 5 to 30, 5 to 20, 10 to 50, 10 to 30, 10 to 20, 15 to 50, 15 to 30, 15 to 20, 20 to 50, and 20 to 30.

### <<(2) Length of spacer sequence>>

By adjusting the length of a spacer sequence of guide RNA, an activity window of a base editor can be controlled. For example, increasing the length of the spacer sequence can expand the activity window of the base editor to the 5' side relative to the nucleic acid strand containing the target sequence. For example, decreasing the length of the spacer sequence can shift the activity window of the base editor to the 3' side relative to the nucleic acid strand containing the target sequence. Alternatively, decreasing the length of the spacer sequence can narrow the activity window of the base editor.

The length of the spacer sequence can be adjusted, for example, within a range of 15 to 50 nucleotide residues. The range within which the length of the spacer sequence is adjusted may be 17 to 50 nucleotide residues, 17 to 40 nucleotide residues, or 17 to 30 nucleotide residues.

### <<(3) Introduction of mismatched nucleotide residues in spacer sequence>>

By introducing mismatched nucleotide residues into the spacer sequence of guide RNA, an activity window of a base editor can be controlled. For example, when a nucleotide residue to be avoided for base editing (hereinafter also referred to as a "non-target nucleotide residue") exists within a target region, a nucleotide residue in the spacer sequence corresponding to the non-target nucleotide residue (hereinafter also referred to as a "non-target corresponding nucleotide residue") can be designed as a mismatched nucleotide residue. This suppresses base editing by the base editor on the non-target nucleotide residue.

The mismatched nucleotide residue is preferably introduced on the 5' side of the 15th nucleotide residue from the 3' end when numbering the nucleotide residues of the spacer sequence from the 3' end toward the 5' side of the spacer sequence. This allows suppression of base editing on the non-target nucleotide residue while avoiding a decrease in the base editing activity of the base editor on the target nucleotide residue. The mismatched nucleotide residue is preferably introduced, for example, further toward the 5' side than the 16th, 17th, 18th, 19th, or 20th nucleotide residue from the 3' end of the spacer sequence toward the 5' side.

The mismatched nucleotide residue may be introduced into a nucleotide residue other than the non-target corresponding nucleotide residue. For example, nucleotide residues around the non-target corresponding nucleotide residue may be designed as mismatched nucleotide residues. For example, the mismatched nucleotide residue may be introduced into one or more nucleotide residues within three nucleotide residues from the non-target corresponding nucleotide residue. In some embodiments, the mis-nucleotide residue is introduced into one or two nucleotide residues adjacent to the non-target corresponding nucleotide residue. In some embodiments, the mismatched nucleotide residue is introduced into the non-target corresponding nucleotide residue and one or more nucleotide residues within three nucleotide residues from the non-target corresponding nucleotide residue. In some embodiments, the mismatched nucleotide residue is introduced into the non-target corresponding nucleotide residue and one or two nucleotide residues adjacent to the non-target corresponding nucleotide residue.

Examples of the number of mismatched nucleotide residues include 0 to 3. By setting the number of mismatched nucleotide residues to 3 or less, preferably 2 or less, base editing in an off-target region can be suppressed. The number of mismatched nucleotide residues is preferably 0 to 2, and may be 0 to 1.

By adjusting one or more of the above-described (1) to (3) to design guide RNA, the width and/or position of the activity window of the base editor can be controlled. Any one of the above-described (1) to (3) may be adjusted alone, or two or more of the above-described (1) to (3) may be adjusted.

### <Expression vector for guide RNA>

An expression vector for guide RNA the same as that described in the above [Method for controlling transcription of guide RNA] can be used.

### <Base editor>

Any known base editor can be used without any particular limitation. Examples of base editors include adenine base editors (which convert adenine to guanine or inosine; ABE, ABE8e, and the like), cytosine base editors (which convert cytosine to thymine; Target-AID, BE4max, and the like), and adenine transversion editors (which convert adenine to cytosine; alkyladenine DNA glycosylase/nCaas9/deaminase TadA-8e).

### <Expression vector for base editor>

An expression vector for a base editor is an expression vector capable of expressing a base editor when introduced into a cell. The expression vector for a base editor preferably includes a coding sequence for the base editor (base editor coding sequence) and a promoter that controls the expression of the base editor coding sequence. In the expression vector for a base editor, the base editor coding sequence is functionally linked to the promoter.

The expression vector for a base editor can have the same configuration as the expression vector for an RNA-guided nuclease described in the above [Method for controlling transcription of RNA], except that it includes the base editor coding sequence instead of the RNA-guided nuclease coding sequence.

### <Expression vector for guide RNA and base editor>

When using an expression vector for a base editor, an expression vector for guide RNA and an expression vector for a base editor may be the same expression vector. In some embodiments, the expression vector is an expression vector capable of expressing guide RNA and a base editor (hereinafter also referred to as a "guide RNA/base editor expression vector"). The guide RNA/base editor expression vector includes a guide RNA coding sequence and a base editor coding sequence, each in an expressible state. The guide RNA/base editor expression vector includes a guide RNA coding sequence functionally linked to a promoter for guide RNA and a base editor coding sequence functionally linked to a promoter for a base editor. A terminator for guide RNA may be linked to the 3' side of the guide RNA coding sequence. A terminator for a base editor coding sequence may be linked to the 3' side of the base editor coding sequence.

The expression vector for guide RNA and a base editor may further include other components. Examples of other components include the same ones described above.

### <Introduction step>

The method of the present embodiment includes: a step of introducing, into a cell, (A) at least one selected from the group consisting of (a1) guide RNA in which one or more selected from the group consisting of the above-described (1) to (3) are adjusted, and (a2) an expression vector for the guide RNA of (a1); and (B) at least one selected from the group consisting of a base editor, mRNA of the base editor, and an expression vector for the base editor.

The introduction step can be performed in the same manner as in the <Introduction step> described in the above [Method for controlling transcription of RNA]. This step may be performed in vitro, ex vivo, or in vivo.

### <Other steps>

The method of the present embodiment may include other steps in addition to the above-described introduction step. Examples of other steps include a step of culturing cells and a step of performing sequence analysis of a region including a target sequence.

The step of culturing a cell can be performed in the same manner as in the <<Culture step>> described in the above [Method for controlling transcription of RNA].

The step of performing sequence analysis of a region including a target sequence can be performed by introducing the above-described (A) and (B) into a cell, followed by analyzing the sequence of the region including the target sequence in the cell. The sequence analysis can be performed by known methods such as Sanger sequencing and next-generation sequencing. The activity window of the base editor can be estimated from the results of the sequence analysis. Therefore, the guide RNA can be redesigned by adjusting the above-described (1) to (3) so that the activity window of the base editor has a desired width and position.

According to the base editing method of the present embodiment, the activity window of the base editor can be controlled by a simple method of adjusting one or more of (1) to (3) in the guide RNA, allowing base editing to be performed more precisely.

### [Guide RNA, vector, and kit]

A fifth aspect of the present disclosure is a kit for controlling transcription of a target gene. The transcription control kit includes: (A) at least one selected from the group consisting of (a1) guide RNA to which one or more nucleotide residues are added at the 5' end, and (a2) an expression vector for the guide RNA of (al); and (B) at least one selected from the group consisting of a fusion protein including an RNA-guided nuclease with inactivated nuclease activity and a transcription regulation-related domain, an mRNA of the fusion protein, and an expression vector for the fusion protein. The guide RNA targets a transcription regulation region of the target gene.

In the transcription control kit according to the fifth aspect, the guide RNA, the expression vector for the guide RNA, the fusion protein, and the expression vector for the fusion protein are the same as those described in the above [Method for controlling transcription of target gene]. Examples of the RNA-guided nuclease with inactivated nuclease activity include Cas protein having no nuclease activity, and dCas is preferred. When the guide RNA is guide RNA of a CRISPR/Cas system, the added nucleotide residues are added to the 5' end of crRNA.

A sixth aspect of the present disclosure is a base editing kit. The base editing kit includes: (A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are controlled, and (a2) an expression vector for the guide RNA of (al); and (B) at least one selected from the group consisting of a base editor, mRNA of the base editor, and an expression vector for the base editor.

In the base editing kit according to the sixth aspect, the guide RNA, the expression vector for the guide RNA, the base editor, and the expression vector for the base editor are the same as those described in the above [Base editing method]. The base editor preferably utilizes nCas. When the guide RNA is guide RNA of a CRISPR/Cas system, the added nucleotide residues are added to the 5' end of crRNA.

A seventh aspect of the present disclosure is an expression vector capable of expressing: (A) guide RNA to which one or more nucleotide residues are added at the 5' end; and (B) a fusion protein including an RNA-guided nuclease with inactivated nuclease activity and a transcription regulation-related protein.

The expression vector according to the seventh aspect is the same as the guide RNA/fusion protein expression vector described in the above [Method for controlling transcription of target gene]. Examples of the RNA-guided nuclease with inactivated nuclease activity include Cas protein having no nuclease activity, and dCas is preferred. When the guide RNA is guide RNA of a CRISPR/Cas system, the added nucleotide residues are added to the 5' end of crRNA.

An eighth aspect of the present disclosure is an expression vector capable of expressing: (A) guide RNA to which one or more nucleotide residues are added at the 5' end; and (B) a base editing editor.

The expression vector according to the eighth aspect is the same as the guide RNA/base editor expression vector described in the above [Base editing method]. The base editor preferably utilizes nCas. When the guide RNA is guide RNA of a CRISPR/Cas system, the added nucleotide residues are added to the 5' end of crRNA.

A ninth aspect of the present disclosure is guide RNA which has a repeat sequence on the 5' side of a spacer sequence and to which one or more nucleotide residues are added at the 5' end.

The guide RNA according to the ninth aspect is the same as the guide RNA described in the above [Genome editing method]. When the guide RNA is guide RNA of a CRISPR/Cas system, the added nucleotide residues are added to the 5' end of crRNA.

A tenth aspect of the present disclosure is an expression vector capable of expressing the guide RNA according to the ninth aspect.

The expression vector according to the tenth aspect is the same as the expression vector for guide RNA described in the above [Genome editing method]. The expression vector according to the tenth aspect may be the guide RNA/RNA-guided nuclease expression vector described in the above [Genome editing method], or may be a guide RNA/Cas protein expression vector.

An eleventh aspect of the present disclosure is a genome editing kit. The genome editing kit includes: (A) at least one selected from the group consisting of (a1) guide RNA which has a repeat sequence on the 5' side of a spacer sequence and to which one or more nucleotide residues are added at the 5' end, and (a2) an expression vector for the guide RNA of (a1); and (B) at least one selected from the group consisting of an RNA-guided nuclease, mRNA of the RNA-guided nuclease, and an expression vector for the RNA-guided nuclease.

In the genome editing kit according to the eleventh aspect, the guide RNA, the expression vector for the guide RNA, the RNA-guided nuclease, and the expression vector for the RNA-guided nuclease are the same as those described in the above [Genome editing method]. Examples of the RNA-guided nuclease include Cas protein of a Type V CRISPR/Cas system, and Cas12a protein is preferred. When the guide RNA is guide RNA of a CRISPR/Cas system, the added nucleotide residues are added to the 5' end of crRNA. Examples of the guide RNA include guide RNA of a Type V CRISPR/Cas system, and guide RNA for Cas12a protein is preferred.

The kit for controlling transcription of a target gene according to the fifth aspect, the base editing kit according to the sixth aspect, and the genome editing kit according to the eleventh aspect may include other components. Other components are not particularly limited, and examples thereof include instructions for use, and reagents used for introducing the components of (A) and/or (B) into cells.

### [Pharmaceutical composition, treatment or prevention method]

A twelfth aspect of the present disclosure is a pharmaceutical composition.
The pharmaceutical composition includes: (A) at least one selected from the group consisting of (a1) guide RNA to which one or more nucleotide residues are added at the 5' end, and (a2) an expression vector for the guide RNA of (a1); and (B) at least one selected from the group consisting of a fusion protein including an RNA-guided nuclease with inactivated nuclease activity and a transcription regulation-related domain, mRNA of the fusion protein, and an expression vector for the fusion protein.

In the pharmaceutical composition according to the twelfth aspect, the guide RNA, the expression vector for the guide RNA, the fusion protein, and the expression vector for the fusion protein are the same as those described in the above [Method for controlling transcription of target gene]. Examples of the RNA-guided nuclease with inactivated nuclease activity include Cas protein having no nuclease activity, and dCas is preferred. When the guide RNA is guide RNA of a CRISPR/Cas system, the added nucleotide residues are added to the 5' end of crRNA.

The pharmaceutical composition according to the twelfth aspect can be used for treatment or prevention of diseases caused by abnormal expression (overexpression or underexpression) of a specific gene.

A thirteenth aspect of the present disclosure is a pharmaceutical composition. The pharmaceutical composition includes: (A) at least one selected from the group consisting of (a1) guide RNA which has a repeat sequence on the 5' side of a spacer sequence and to which one or more nucleotide residues are added at the 5' end, and (a2) an expression vector for the guide RNA of (a1).

**In** the pharmaceutical composition according to the thirteenth aspect, the guide RNA and the expression vector for the guide RNA are the same as those described in the above [Genome editing method]. The pharmaceutical composition according to the thirteenth aspect may further include: (B) at least one selected from the group consisting of an RNA-guided nuclease, mRNA of the RNA-guided nuclease, and an expression vector for the RNA-guided nuclease. The RNA-guided nuclease and the expression vector for the RNA-guided nuclease are the same as those described in the above [Genome editing method]. Examples of the RNA-guided nuclease include Cas protein of a Type V CRISPR/Cas system, and Cas12a protein is preferred. When the guide RNA is guide RNA of a CRISPR/Cas system, the added nucleotide residues are added to the 5' end of crRNA. Examples of the guide RNA include guide RNA of a Type V CRISPR/Cas system, and guide RNA for Cas12a protein is preferred.

The pharmaceutical composition according to the thirteenth aspect can be used for treatment or prevention of hereditary diseases or the like. Examples of hereditary diseases include cancer, sickle cell anemia, Huntington's disease, Duchenne muscular dystrophy, fibrodysplasia ossificans progressiva (FOP), and gene tyrosinemia type I (HT-1), but are not limited thereto.

A fourteenth aspect of the present disclosure is a pharmaceutical composition. The pharmaceutical composition includes: (A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (a1); and (B) at least one selected from the group consisting of a base editor, mRNA of the base editor, and an expression vector for the base editor.

In the pharmaceutical composition according to the fourteenth aspect, the guide RNA, the expression vector for the guide RNA, the base editor, and the expression vector for the base editor are the same as those described in the above [Base editing method]. The base editor preferably utilizes nCas. When the guide RNA is guide RNA of a CRISPR/Cas system, the added nucleotide residues are added to the 5' end of crRNA.

The pharmaceutical composition according to the fourteenth aspect can be used for treatment or prevention of hereditary diseases caused by SNPs. Examples of hereditary diseases caused by SNPs include cancer, sickle cell anemia, Huntington's disease, Duchenne muscular dystrophy, fibrodysplasia ossificans progressiva (FOP), and gene tyrosinemia type I (HT-1), but are not limited thereto.

The pharmaceutical compositions according to the twelfth to fourteenth aspects may include other components in addition to the above-described components. Examples of other components include pharmaceutically acceptable carriers. The pharmaceutical compositions may be formulated by blending a pharmaceutically acceptable carrier or the like. Examples of pharmaceutically acceptable carriers include excipients, binders, disintegrants, lubricants, colorants, flavoring and odor-masking agents, stabilizers, emulsifiers, absorption promoters, surfactants, pH adjusters, preservatives, and antioxidants.

The administration form of the pharmaceutical compositions is not particularly limited, and the pharmaceutical compositions can be administered orally or parenterally. Examples of forms of parenteral administration include intravenous injection, intravenous drip infusion, subcutaneous injection, intradermal injection, or intraperitoneal injection. The dosage of the pharmaceutical composition can be appropriately set depending on types of diseases, symptoms of diseases, age, sex, body weight, and sensitivity difference of the subject, administration methods, administration timings, administration intervals, administration periods, properties of the formulation, types of active ingredients, and the like.

A fifteenth aspect of the present disclosure is a method for treating or preventing a disease caused by abnormal gene expression. The treatment or prevention method includes: administering, to a subject in need of treatment or prevention, (A) at least one selected from the group consisting of (a1) guide RNA to which one or more nucleotide residues are added at the 5' end, and (a2) an expression vector for the guide RNA of (a1) and (B) at least one selected from the group consisting of a fusion protein including an RNA-guided nuclease with inactivated nuclease activity and a transcription regulation-related domain, mRNA of the fusion protein, and an expression vector for the fusion protein.

In the treatment or prevention method according to the fifteenth aspect, the guide RNA, the expression vector for the guide RNA, the fusion protein, and the expression vector for the fusion protein are the same as those described in the above [Method for controlling transcription of target gene]. Examples of the RNA-guided nuclease with inactivated nuclease activity include Cas protein having no nuclease activity, and dCas is preferred. When the guide RNA is guide RNA of a CRISPR/Cas system, the added nucleotide residues are added to the 5' end of crRNA. The (A) and (B) may be administered simultaneously or separately.

A sixteenth aspect of the present disclosure is a method for treating or preventing a hereditary disease. The treatment or prevention method includes: administering, to a subject in need of treatment or prevention, (A) at least one selected from the group consisting of (a1) guide RNA which has a repeat sequence on the 5' side of a spacer sequence and to which one or more nucleotide residues are added at the 5' end, and (a2) an expression vector for the guide RNA of (a1) and (B) at least one selected from the group consisting of an RNA-guided nuclease, mRNA of the RNA-guided nuclease, and an expression vector for the RNA-guided nuclease.

In the treatment or prevention method according to the sixteenth aspect, the guide RNA, the expression vector for the guide RNA, the RNA-guided nuclease, and the expression vector for the RNA-guided nuclease are the same as those described in the above [Genome editing method]. Examples of the RNA-guided nuclease include Cas protein of a Type V CRISPR/Cas system, and Cas12a protein is preferred. When the guide RNA is guide RNA of a CRISPR/Cas system, the added nucleotide residues are added to the 5' end of crRNA. Examples of the guide RNA include guide RNA of a Type V CRISPR/Cas system, and guide RNA for Cas12a protein is preferred. The (A) and (B) may be administered simultaneously or separately.

A seventeenth aspect of the present disclosure is a method for treating or preventing a hereditary disease. The treatment or prevention method includes: administering, to a subject in need of treatment or prevention, (A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (a1) and (B) at least one selected from the group consisting of a base editor and an expression vector therefor.

In the treatment or prevention method according to the seventeenth aspect, the guide RNA, the expression vector for the guide RNA, the base editor, and the expression vector for the base editor are the same as those described in the above [Base editing method]. The base editor preferably utilizes nCas. When the guide RNA is guide RNA of a CRISPR/Cas system, the added nucleotide residues are added to the 5' end of crRNA. The (A) and (B) may be administered simultaneously or separately.

In the treatment or prevention methods according to the above-described fifteenth to seventeenth aspects, the dosage of the components of (A) and (B) can be a therapeutically effective amount. The therapeutically effective amount is a dosage at which a therapeutic effect or a preventive effect can be exhibited. The therapeutically effective amount can be appropriately set depending on types of diseases, symptoms of diseases, age, sex, body weight, and sensitivity difference of the subject, administration methods, administration timings, administration intervals, administration periods, properties of the formulation, types of active ingredients, and the like.

### Examples

Hereinafter, the present invention will be described in more detail using examples, but is not limited to these examples.

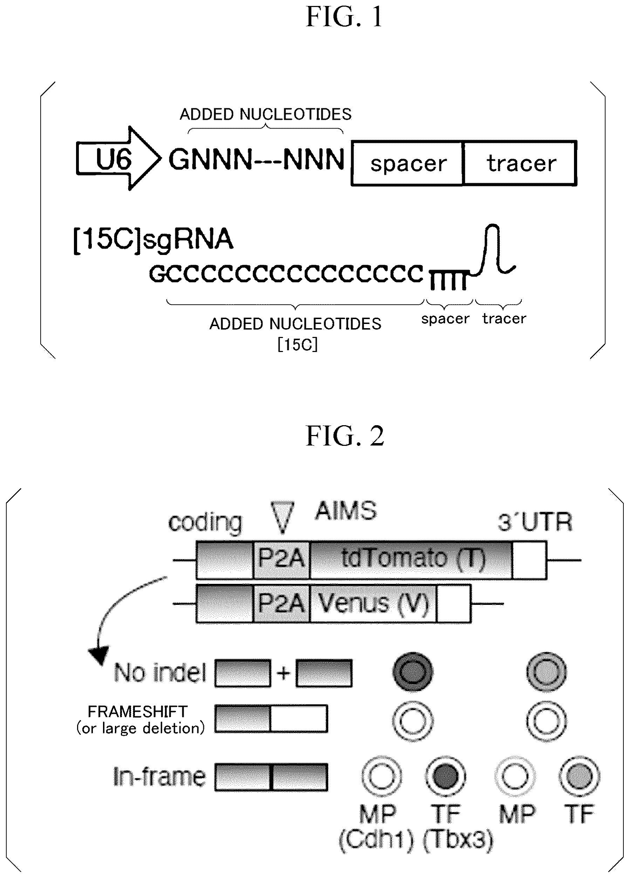

In the following experimental examples, nucleotide residues added to guide RNA are referred to as "added nucleotide residues." Added nucleotide residues added to the 5' end of a spacer are referred to as "5'-added nucleotide residues." For example, in FIG. 1, nucleotide residues indicated by "NNN---NNN" are added nucleotide residues.

When added nucleotide residues consist of the same nucleotide residue, the added nucleotide residues are denoted by attaching the number of nucleotide residues to the left side of an abbreviation indicating the type of nucleotide residues constituting the added nucleotide residues. For example, the sgRNA shown in FIG. 1 has added nucleotide residues consisting of 15 cytosine nucleotide residues. In this case, the added nucleotide residues are denoted as [15C]. Unless otherwise specified, [nN]gRNA represents gRNA having added nucleotide residues consisting of n N nucleotide residues at the 5' end of a spacer. The N represents any type of nucleotide residue, and is an adenine residue (A), a guanine residue (G), a cytosine residue (C), or a thymine residue (T). The notation of [ ] and/or "gRNA" may be omitted in some cases.

The notation [N] represents added nucleotide residues consisting of N nucleotide residues having an arbitrary length. The N represents the type of any nucleotide residue, which is an adenine residue (A), a guanine residue (G), a cytosine residue (C), or a thymine residue (T).

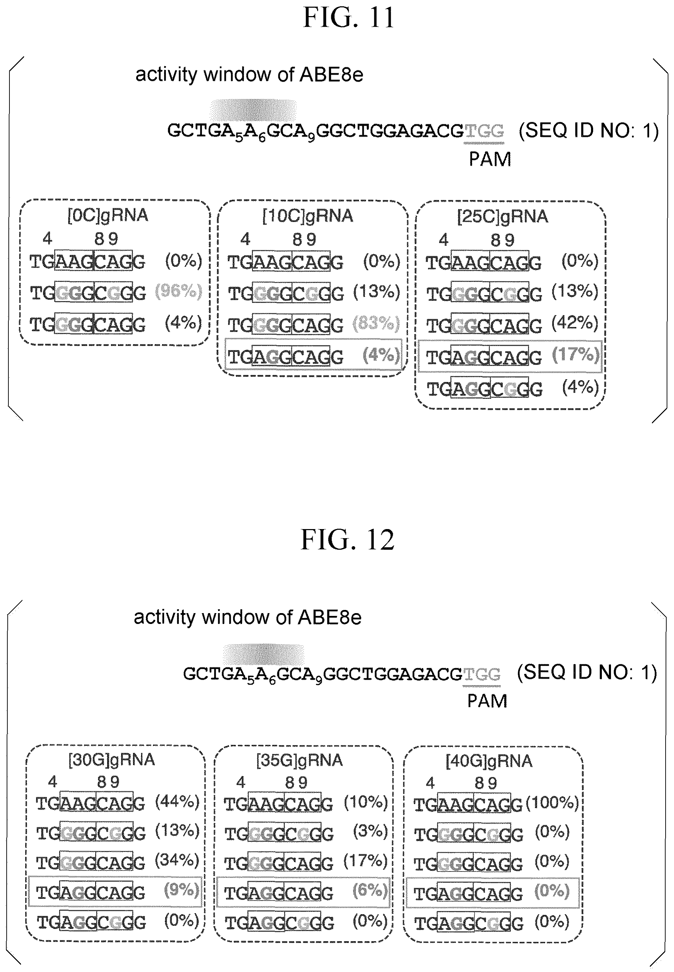

The nucleotide residues constituting the target sequence of gRNA may be denoted by attaching a number in order from the 5' side to the type of base. For example, in the target sequence shown in FIG. 11, "A₅" represents the fifth adenine residue from the 5' side of the target sequence. "A₆" represents the sixth adenine residue from the 5' side of the target sequence. "A₉" represents the ninth adenine residue from the 5' side of the target sequence.

### <Experimental Example 1>

### (Culture of mouse ES cells)

For culturing mouse ES cells (mESCs), t2iL medium was used. The t2iL medium is a medium containing Dulbecco's Modified Eagle Medium (DMEM, Nacalai Tesque), 2 mM Glutamax (Nacalai Tesque), 1x non-essential amino acids (Nacalai Tesque), 1 mM sodium pyruvate (Nacalai Tesque), 100 U/mL penicillin, 100 µg/mL streptomycin (P/S) (Nacalai Tesque), 0.1 mM 2-mercaptoethanol (Sigma), and 15% fetal bovine serum (FBS) (Gibco), to which 0.2 µM PD0325901 (Sigma), 3 µM CHIR99021 (Cayman), and 1,000 U/mL recombinant mouse leukemia inhibitory factor (LIF) (Millipore) were added. For the 2iL medium, 1 µM PD0325901 was used. mESC colonies were dissociated with trypsin (Nacalai Tesque) and plated on gelatin-coated dishes. When passaging the cells, Y-27632 (10 µM, Sigma) was added.

### (Construction of plasmids)

To prepare all-in-one CRISPR plasmids for expression of sgRNA of [5C] (3A), [10C] (8A), [15C] (13A), [20C] (18C), [25C] (23A), and [30C] (28A), respectively, each spacer linker was inserted into the BpiI site of the PX459 plasmid. In this plasmid, since the CACC overhang sequence is necessary for linker ligation, the 3rd, 8th, 13th, 18th, 23rd, or 28th cytosine was substituted with adenine. A standard spacer linker (20 nt) was inserted into the BpiI site of the [0C], [5C] (3A), [10C] (8A), [15C] (13A), [20C] (18A), [25C] (23A), or [30C] (28A) PX459 plasmids, or a long spacer linker (30 nt or 40 nt) was inserted into the BpiI site of the PX459 plasmids. This allowed preparation of all-in-one Cas9 plasmids for expression of sgRNAs of [5C] to [30C] applicable to puromycin selection.

### (Northern blotting)

Total RNA was extracted from mESCs 68 hours after transfection with the P2A₁-[C]sgRNA1-PX459 plasmid. The transfected cells were selected by treatment with puromycin (1.5 µg/mL) for 2 days and resuspended in ISOGEN II (NIPPON GENE). The samples were incubated at room temperature for 10 minutes and then heated at 55°C for 10 minutes. Total RNA was isolated according to the manufacturer's protocol. After reaction at 70°C for 10 minutes, 30 µg of RNA was loaded onto Extra PAGE One Precast Gels (5-20%) (Nacalai Tesque) in Tris-borate-EDTA buffer. RNA was transferred to a Hybond N+ membrane (GE Healthcare) and crosslinked using CX-2000 (Analytik Jena). The sgRNA tracer probe was labeled with alkaline-labile digoxigenin (DIG)-11-deoxyuridine triphosphate (dUTP) using a PCR DIG Probe Synthesis Kit (Roche). After hybridization, specific bands were visualized using CDP-Star reagent (Roche) with a luminescent image analyzer (LAS-3000, FUJIFILM). The DIG probe was labeled by PCR amplification of a DNA fragment. The mU6 DIG-probe was prepared by amplifying a DNA fragment from mESC cDNA using specific primers. cDNA was synthesized using specific primers targeting U6 snRNA (Ranganathan, V., Wahlin, K., Maruotti, J. & Zack, D. J. Nat Commun 5, 4516, doi:10.1038/ncomms5516 (2014).).

### (Measurement of sgRNA expression level)

The effect of the number of 5'-added nucleotide residues on the expression level of sgRNAs was investigated. All-in-one Cas9 plasmids for expression of sgRNAs of [0C] to [30C] were introduced into mouse ES cells, respectively. At 68 hours after transfection, total RNA was extracted from each mESC, and the expression level of the sgRNAs was analyzed by Northern blot analysis.

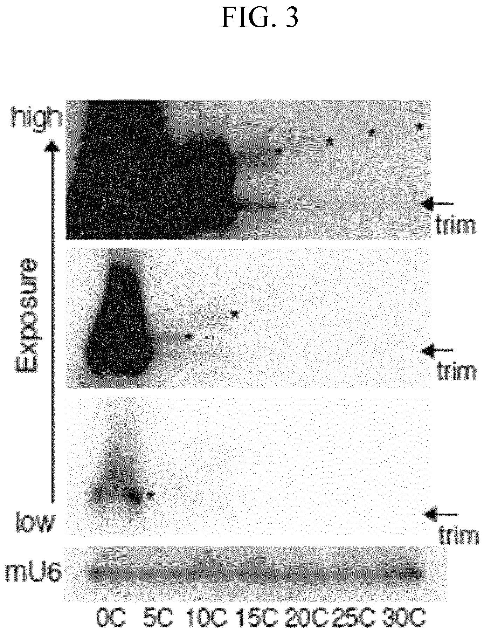

The results are shown in FIG. 3. The expression level of the sgRNAs decreased as the number of 5'-added nucleotide residues increased. In the figure, the asterisks indicate sgRNAs having 5'-added nucleotide residues. "trim" indicates sgRNA in which the 5'-added nucleotide residues have been trimmed.

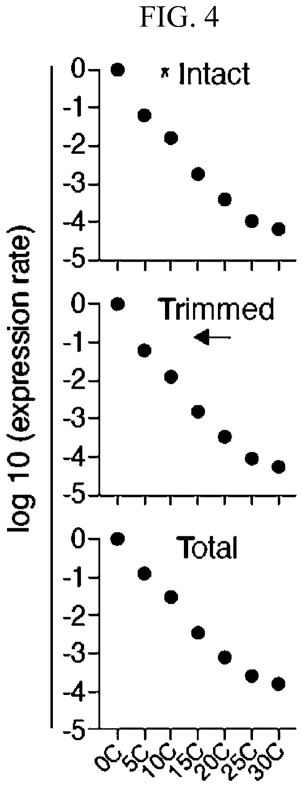

FIG. 4 shows the quantified results of the signal from the Northern blot analysis in FIG. 3. It was confirmed that the logarithm of the expression level of the sgRNAs has a negative correlation with the number of 5'-added nucleotide residues. From these results, it was confirmed that the expression level of the sgRNAs can be adjusted by the number of 5'-added nucleotide residues.

### <Experimental Example 2>

### (Culture of HEK293T cells)

HEK293T cells were cultured in 10% FBS medium. The 10% FBS medium is a medium containing DMEM, 2 mM L-glutamine (Nacalai Tesque), 100 U/mL penicillin, 100 µg/mL streptomycin (P/S) (Nacalai Tesque), and 10% FBS. HEK293T cells were maintained at 37°C and 5% CO2.

### (Measurement of sgRNA expression level)

The effect of the type of 5'-added nucleotide residues on the expression level of sgRNA was investigated. All-in-one Cas9 plasmids for expression of sgRNAs of [15G], "25G," [15A], "25A," [15T], [15hp], and [15s] were constructed. [15hp] is sgRNA having added nucleotide residues of 15 nucleotide residues and forming a hairpin structure in sgRNA. [15s] is sgRNA having 5'-added nucleotide residues of 15 nucleotides and not forming a hairpin structure in sgRNA. All-in-one Cas9 plasmids for expression of sgRNAs of [0C] to [30C], [15G], "25G," [15A], "25A," [15T], [15hp], and [15s] were introduced into HEK293 cells, respectively. At 64 hours after transfection, total RNA was extracted from each HEK293 cell, and the expression level of the sgRNAs was analyzed by Northern blot analysis.

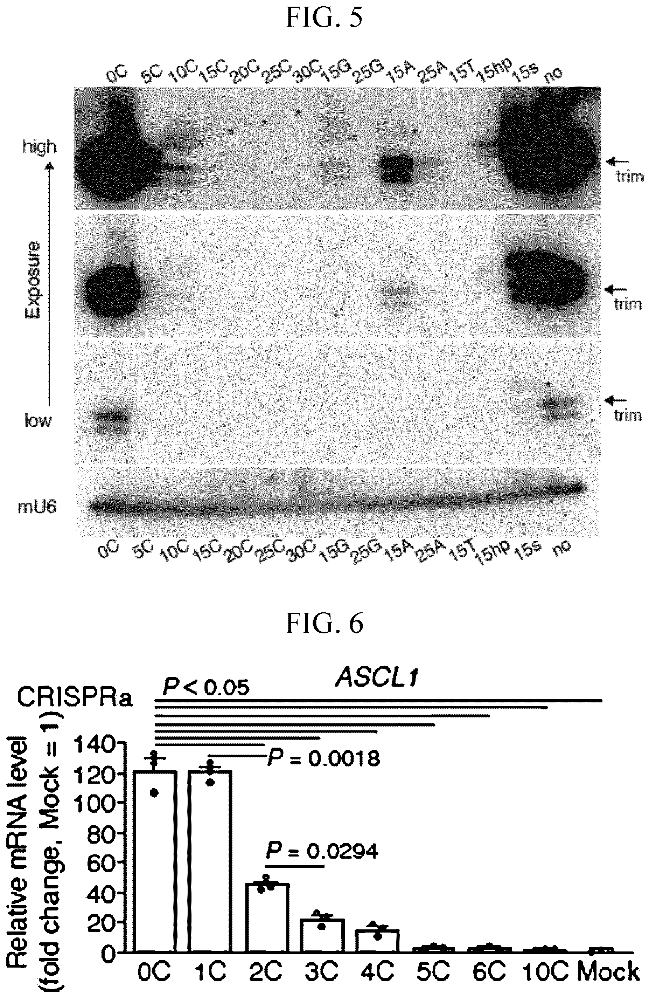

The results are shown in FIG. 5. The expression level of the sgRNAs decreased as the number of 5'-added nucleotide residues increased. Among [C], [A], [G], and [T], [C] had the highest sgRNA expression suppression effect. The gRNA expression suppression effect was also confirmed in 15hp. From these results, it was confirmed that the expression level of the sgRNAs can be consistently adjusted by the number of 5'-added nucleotide residues, regardless of the type of nucleotide residues.

By regulating the expression of the RNAs, it is considered that the amount of excessive gRNAs that could be toxic in cells can be reduced, enabling safe genome editing. In addition, by regulating the expression of the gRNAs, it is considered that genome editing activity by an RNA-guided nuclease can be controlled. In addition, by regulating the expression of the guide RNAs, it is considered that genome editing at off-target sites by the RNA-guided nuclease can be suppressed. In addition, by regulating the expression of the guide RNAs, it is considered that induction of unnecessary mutations such as bystanders by a base editor can be suppressed, enabling safe medical applications.

In addition to the conventional regulation of RNA expression by adjusting promoters, it was confirmed that RNA expression regulation can be achieved by adjusting the length of added nucleotide residues.

### <Experimental Example 3>

### (Construction of plasmids for CRISPRa)

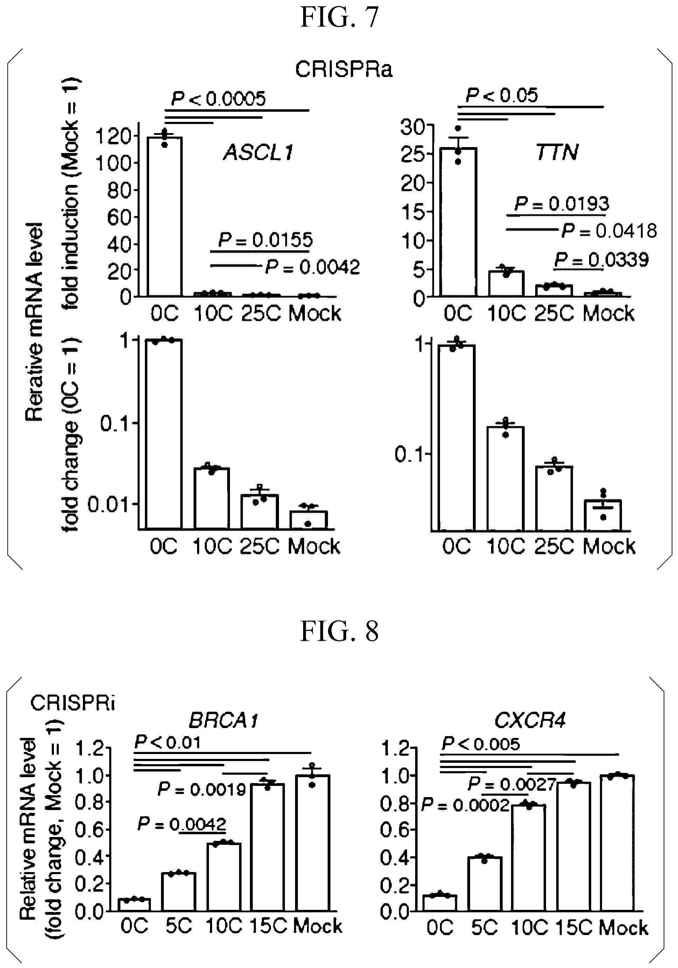

A puromycin-selectable all-in-one plasmid for CRISPRa was constructed by replacing the GFP cassette in the pLV hU6-gRNA (antisense) hUbC-VP64-dCas9-VP64-T2A-GFP (sgRNA-VP64-GFP) plasmid (Addgene #66707) with a puromycin N-acetyltransferase (PuroR) cassette. A synthetic gene encoding VP64-T2A-PuroR (AZENTA) was inserted into the sgRNA-KRAB-GFP plasmid using Nhel and Agel sites to obtain sgRNA-VP64-Puro plasmids. In FIG. 6, spacer linkers of [1C] to [10C] targeting ASCL1 (Chavez, A. et al. Nat Methods 13, 563-1240 567, doi:10.1038/nmeth.3871 (2016).) were inserted into the sgRNA-VP64-Puro plasmids, respectively. In FIG. 7, spacer linkers targeting ASCL1 and TTN (Chavez, A. et al. Nat Methods 13, 563-1240 567, doi:10.1038/nmeth.3871 (2016).) were inserted into the Bpil site of the sgRNA expression plasmids of [0C] to [30C], respectively. Subsequently, co-transfection was performed with an all-in-one CRISPRa plasmid without a spacer.

### (qRT-PCR)

cDNA was synthesized from total RNA using SuperScript III Reverse Transcriptase (Thermo Fisher Scientific) according to the manufacturer's instructions. qRT-PCR was performed using THUNDERBIRD (registered trademark) SYBR qPCR Mix (Toyobo) and the CFX Connect real-time PCR detection system (BIO RAD) according to the manufacturer's instructions. The value of GAPDH was used as a normalization control.

### (Transcription activation test by CRISPRa)

Whether transcriptional activity by CRISPRa can be regulated by 5'-added nucleotide residues was investigated. The day before transfection, 3×10⁴ HEK293T cells were seeded in a 96-well plate. All-in-one CRISPRa plasmids (50 ng, 1/5 scale of a 24-well plate) respectively having 5'-added nucleotide residues of [0C] to [25C] were transfected into the cells, and the transfected cells were cultured for 24 hours. Subsequently, the cells were treated with puromycin (5.0 µg/mL) for 2 days to remove untransfected cells. After removing puromycin, the transfected cells were cultured for 1 day, and total RNA was extracted using ISOGEN II. Then, the mRNA expression levels of ASCL1 and TTN were measured by qRT-PCR.

The results are shown in FIGS. 6 and 7. The mRNA expression levels of both ASCL1 and TTN decreased as the number of 5'-added nucleotide residues increased. From these results, it was confirmed that the transcriptional activity of CRISPRa can be regulated by the number of 5'-added nucleotide residues.

### <Experimental Example 4>

### (Construction of plasmids for CRISPRi)

Linkers of [5C] to [30C] containing a BmsBI site were inserted into the BsmBI site of the LV hU6-sgRNA hUbC-dCas9-KRAB-T2a-Puro (sgRNA-KRAB-Puro) plasmid (Addgene #71236) to construct all-in-one CRISPRi plasmids for [C] sgRNA expression. sgRNA spacers targeting BRCA1 and CXCR4, used in previous studies, were inserted into the BsmBI site of the all-in-one plasmids (Yeo, N. C. et al. Nat Methods 15, 611-616, doi:10.1038/s41592-018-0048-5 (2018).).

### (Transcription suppression test by CRISPRi)

Whether transcription suppression by CRISPRi can be regulated by 5'-added nucleotide residues was investigated. The day before transfection, 3×10⁴ HEK293T cells were seeded in a 96-well plate. All-in-one CRISPRi plasmids (50 ng, 1/5 scale of a 24-well plate) respectively having 5'-added nucleotide residues of [0C] to [15] were transfected into the cells, and the transfected cells were cultured for 24 hours. Subsequently, the cells were treated with puromycin (5.0 µg/mL) for 2 days to remove untransfected cells. After removing puromycin, the transfected cells were cultured for 2 days, and total RNA was extracted using ISOGEN II. Then, the mRNA expression levels of BRCA1 and CXCR4 were measured by qRT-PCR.

The results are shown in FIG. 8. The mRNA expression levels of both BRCA1 and CXCR4 increased as the number of 5'-added nucleotide residues increased. From these results, it was confirmed that the transcription suppression effect by CRISPRi decreases as the number of 5'-added nucleotide residues increases, and the activity of CRISPRi can be regulated by the number of 5'-added nucleotide residues.

In this manner, it was revealed that the activity of the CRISPR/Cas system also decreases as the number of 5'-added nucleotide residues increases. In the CRISPRi system, the amount of transcriptional activation has been regulated by changing the type of transcription activation domain, but it was suggested that the degree of transcriptional activation can be finely adjusted by changing the length of 5'-added nucleotide residues without changing the transcription activation domain.

### <Experimental Example 5>

### (Preparation of AIMS cells)

AIMS cells are cells capable of detecting indel introduction patterns caused by genome editing by detecting fluorescence of cells after genome editing (refer to FIG. 2). The preparation of AIMS cells was performed based on the method described in PCT International Publication No. WO2020/122195. AIMS cells (Cdh1-AIMS) were prepared by knocking in a P2A₂:Venus cassette and a P2A₂:tdTomato cassette into the region adjacent to the 3' side of the Cdh1 coding region of mouse ES cells.

### (Construction of plasmids for Cas12a)

A synthetic DNA fragment encoding a U6 promoter and two BpiI sites (AZENTA) was inserted into the PX459 plasmid using Pcil and Xbal sites while removing the U6-gRNA cassette. Next, the CBh-Cas9 region of the crRNA-Cas9-puro plasmid was replaced with a CBh-AsCpf1 fragment digested from the pY036_ATP1A1_G3_Array plasmid (Addgene #86619) (Agudelo, D. et al. Nat Methods 14, 615-620, doi:10.1038/nmeth.4265 (2017).) using KpnI and Fsel. This allowed construction of an all-in-one crRNA-AsCpf1-puro plasmid (PX459 plasmid backbone). A crRNA linker targeting the P2A₂ site of Cdh1-AIMS consists of a 5' hairpin, a 20 nt spacer, and a U₄AU₄ 3'-overhang. These were inserted into the Bpil site of the crRNA-AsCpf1-puro plasmid.

### (Indel induction test by Cas12a platform)

Whether the indel induction rate in the Cas12a platform can be regulated by added nucleotide residues was investigated. The position of the added nucleotide residues was set at (1) the 5' end of sgRNA, (2) the 5' end of the spacer, or (3) the 3' end of sgRNA. All-in-one plasmids for AsCpf1 having added nucleotide residues of [10C] or [25C] at any one of the positions (1) to (3) were prepared. These all-in-one plasmids for Cas12a were introduced into Cdh1-AIMS. Subsequently, indel patterns induced by Cas12a genome editing were confirmed using fluorescence microscopes (BZ-X800, Keyence, and IX73, Olympus).

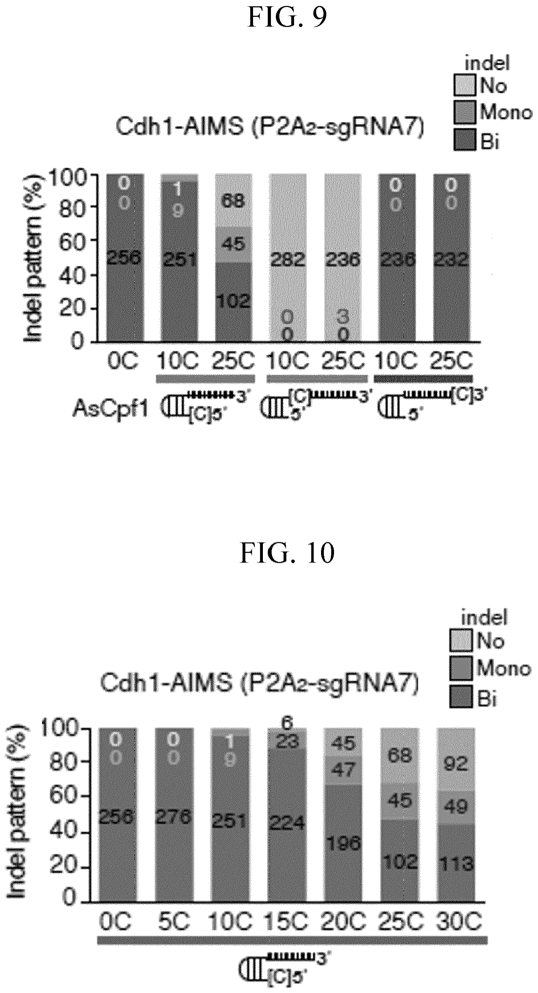

The results are shown in FIG. 9. When the position of the added nucleotide residues was set at (1) the 5' end of sgRNA, the indel induction rate decreased as the number of added nucleotide residues increased. In addition, the indel induction rate into only one allele increased. From these results, it is considered that genome editing activity is reduced by the added nucleotide residues, and an off-target suppression effect can be obtained. This suggests that genome editing in one allele can be efficiently induced.

When the position of the added nucleotide residues was set at (2) between the repeat sequence and the spacer sequence, no indels were introduced.

When the position of the added nucleotide residues was set at (3) the 3' end of sgRNA, the added nucleotide residues did not affect the indel induction rate.

From the above-described results, it was confirmed that in the Cas12a platform, the genome editing activity of Cas12a can be regulated by adding added nucleotide residues to the 5' end of sgRNA. Therefore, the effect of the number of added nucleotide residues was investigated using the all-in-one plasmids for Cas12a having added nucleotide residues of [0C] to [30C] at the 5' end of sgRNA.

The results are shown in FIG. 10. The indel induction rate decreased as the number of added nucleotide residues increased. In addition, the indel induction rate into only one allele increased as the number of added nucleotide residues increased. From these results, it was confirmed that in the Cas12a platform, genome editing activity can be regulated by adjusting the number of added nucleotide residues added to the 5' end of sgRNA. By suppressing genome editing activity, an off-target suppression effect can be expected. By combining with HDR, this can be applied to preparation of disease model cells and disease model animals having heterozygous SNPs without unnecessary mutations. It is also considered useful for implementing precise gene therapy for diseases developed in association with heterozygous SNPs.

### <Experimental Example 6>

### (Culture of mESC)

mESCs were cultured in DMEM (Nacalai Tesque). The DMEM used for culture contained 2 mM Glutamax (Nacalai Tesque), 1x non-essential amino acids (NEAA) (Nacalai Tesque), 1 mM sodium pyruvate, 100 U/mL penicillin, 100 µg/mL streptomycin (P/S) (Nacalai Tesque), 0.1 mM 2-mercaptoethanol (Sigma), and 15% fetal bovine serum (FBS) (GIBCO), with the addition of 0.2 µM PD0325901 (Sigma), 3 µM CHIR99021 (Cayman), and 1,000 U/mL recombinant mouse LIF (Millipore). mESCs were maintained under feeder-free conditions at 37°C and 5% CO₂. When passaging the cells, Y-27632 (10 µM, Sigma) was added to the medium.

### (Base substitution test by ABE8e)

Cdh1-AIMS, in which P2A sequences along with tdTomato and Venus were knocked into both alleles at the end of the Cdhl gene coding sequence (refer to FIG. 1; Kawamata et al., Nat. Biomed. Eng. 7, 672-691 (2023), PCT International Publication No. WO2020/122195), was used. An ABE8e all-in-one plasmid expressing P2A-sgRNA3 targeting the P2A-3 site (spacer: GCTGAAGCAGGCTGGAGACGTGG; SEQ ID NO: 1; the 3' end "TGG" is the PAM sequence) was constructed. This was transfected into Cdh1-AIMS to induce base substitution.

The ABE8e all-in-one plasmid (SEQ ID NO: 2) is a plasmid expressing all of ABE8e, a puromycin resistance cassette, and gRNA. The ABE8e plasmid (Addgene, Plasmid #138489) (SEQ ID NO: 3) was cleaved with SpeI and BglII to excise a fragment. The pSpCas9(BB)-2A-Puro (PX459) V2.0 plasmid (Addgene, Plasmid #62988) (SEQ ID NO: 4) was cleaved with Xbal and BglII, and the fragment was ligated. This allowed preparation of the ABE8e all-in-one plasmid.

The following adapter linkers were inserted into the Bpil site immediately downstream of the U6 promoter of the ABE8e all-in-one plasmid. (F) indicates a forward linker. (R) indicates a reverse linker.

5C (3A) Adapter linkers:
   (F) 5'-CACCGCCACCGGGTCTTCGAGAAGACCT-3' (SEQ ID NO: 5)
   (R) 5'-AAACAGGTCTTCTCGAAGACCCGGTGGC-3' (SEQ ID NO: 6)
10C (8A) Adapter linkers:
   (F) 5'-CACCGCCCCCCCACCGGGTCTTCGAGAAGACCT-3' (SEQ ID NO: 7)
   (R) 5'-AAACAGGTCTTCTCGAAGACCCGGTGGGGGGGC-3' (SEQ ID NO: 8)
15C (13A) Adapter linkers:
   (F) 5'-CACCGCCCCCCCCCCCACCGGGTCTTCGAGAAGACCT-3' (SEQ ID NO: 9)
   (R) 5'-AAACAGGTCTTCTCGAAGACCCGGTGGGGGGGGGGGC-3' (SEQ ID NO: 10)
20C (18A) Adapter linkers:
   (F) 5'-CACCGCCCCCCCCCCCCCCCACCGGGTCTTCGAGAAGACCT-3' (SEQ ID NO: 11)
   (R) 5'-AAACAGGTCTTCTCGAAGACCCGGTGGGGGGGGGGGGGGGC-3' (SEQ ID NO: 12)
25C (23A) Adapter linkers:
   (F) 5'-CACCGCCCCCCCCCCCCCCCCCCCACCGGGTCTTCGAGAAGACCT-3' (SEQ ID NO: 13)
   (R) 5'-AAACAGGTCTTCTCGAAGACCCGGTGGGGGGGGGGGGGGGGGGGC-3' (SEQ ID NO: 14)
30C (28A) Adapter linkers:
   (F) 5'-CACCGCCCCCCCCCCCCCCCCCCCCCCCACCGGGTCTTCGAGAAGACCT-3' (SEQ ID NO: 15)
   (R) 5'-AAACAGGTCTTCTCGAAGACCCGGTGGGGGGGGGGGGGGGGGGGGGGGC-3' (SEQ ID NO: 16)

This allowed preparation of 5C (3A) linker ABE8e all-in-one, 10C (8A) linker ABE8e all-in-one, 15C (13A) linker ABE8e all-in-one, 20C (18A) linker ABE8e all-in-one, 25C (23A) linker ABE8e all-in-one, and 30C (28A) linker ABE8e all-in-one, respectively. By inserting a coding sequence for a desired spacer sequence into the Bpil site of these plasmids, sgRNAs having 5C, 10C, 15C, 20C, 25C, and 30C added to the 5' side can be expressed, respectively. However, in these plasmids, to introduce the overhang sequence CACC for ligation into the Bpil site, the 3rd C of 5C, the 8th C of 10C, the 13th C of 15C, the 18th C of 20C, the 23rd C of 25C, and the 28th C of 30C are each substituted with A.

A coding sequence for a spacer targeting P2A-3 was inserted into the BpiI site of the ABE8e all-in-one plasmids into which the above linkers were inserted. In addition, a coding sequence for a spacer targeting P2A-3 was inserted into the BpiI site of the ABE8e all-in-one plasmid into which no linker was inserted. This allowed preparation of P2A-sgRNA3_ABE8e all-in-one (0C), P2A-sgRNA3_ABE8e all-in-one (5C (3A)), P2A-sgRNA3_ABE8e all-in-one (10C (8A)), P2A-sgRNA3_ABE8e all-in-one (15C (13A)), P2A-sgRNA3_ABE8e all-in-one (20C (18A)), P2A-sgRNA3_ABE8e all-in-one (25C (23A)), and P2A-sgRNA3_ABE8e all-in-one (30C (28A)), respectively. The nucleotide sequences of the linkers for preparing sgRNA targeting P2A-3 are shown in SEQ ID NOs: 17 and 18.

The plasmids prepared as described above were introduced into Cdh1-AIMS using Lipofectamine (registered trademark) 3000 (Thermo Fisher Scientific). 500 µL of 2iL+Y medium was dispensed into a collagen-coated 24-well plate. ES cells separated with trypsin (Nacalai Tesque) were seeded therein. Nucleic acid-Lipofectamine 3000 complexes were prepared according to the standard protocol of Lipofectamine 3000. 1 µL of Lipofectamine 3000 was added to 25 µL of Opti-MEM medium (Thermo Fisher Scientific). In addition, 250 ng of the plasmid and 1 µL of a P3000 reagent were added to another 25 µL of Opti-MEM medium and mixed. These mixtures were mixed together, and incubated at room temperature for 5 minutes. Thereafter, the mixture was added to the 24-well plate seeded with ES cells and incubated overnight. After 1 day of transfection, the cells were treated with puromycin (1.5 µg/mL) for 2 days. Puromycin-resistant cells selected by puromycin treatment were cultured for several days in the absence of puromycin, trypsinized, and reseeded on collagen-coated plates.

After several days of culture, emerged colonies were picked up. Using DNA of the picked colonies as a template, the region including the target sequence of the Cdh1-P2A-tdTomato chimeric gene (SEQ ID NO: 19) was amplified by PCR. Then, the nucleotide sequence of the amplified DNA fragment was obtained by Sanger sequencing. The base editing pattern was identified by comparing the obtained nucleotide sequence with the Cdh1-P2A-tdTomato chimeric gene (SEQ ID NO: 19). The PCR primer sequences for clone sequence analysis are shown in SEQ ID NOs: 20 and 21.

The results are shown in FIG. 11. With [0C] gRNA, no clones in which only the active center (A₆) was substituted with a guanine residue (A₆>G) were detected, and 96% of the clones had all of A₅, A₆, and A₉ substituted with guanine.

On the other hand, with [10C] gRNA and [25C] gRNA, clones in which only the active center (A₆) was substituted with guanine (A₆>G) were detected. In addition, the percentage of clones in which all of A₅, A₆, and A₉ were substituted with guanine decreased. With [25C] gRNA, clones in which A₆ and A₉ were substituted with guanine appeared at 4%.

From these results, it was confirmed that the width of the activity window of ABE8e can be adjusted by adjusting the number of 5'-added nucleotide residues. It is considered that by adjusting the number of 5'-added nucleotide residues, adjustment of the width of the activity window at the 1 bp level is possible. That is, it can be said that 5'-added nucleotide residues have an effect of suppressing the bystander effect of ABE8e. Furthermore, it was confirmed that by adjusting the number of 5'-added nucleotide residues, movement of the position of the activity window is also possible.

### <Experimental Example 7>

The same test as in Experimental Example 6 was performed using [30G] gRNA, [35G] gRNA, and [40G] gRNA. P2A-sgRNA3_ABE8e all-in-one (30G), P2A-sgRNA3_ABE8e all-in-one (35G), and P2A-sgRNA3_ABE8e all-in-one (40G) were prepared in the same manner as described above, except that the linker for preparing sgRNA was changed to linkers for preparing sgRNA for [30G], [35G], and [40G] (SEQ ID NOs: 24 to 29), respectively. Using these plasmids, a base substitution test and base substitution analysis were performed in the same manner as in Experimental Example 6. The same PCR primers for clone sequence analysis as in Experimental Example 6 were used.

The results are shown in FIG. 12. With [30G] gRNA and [35G] gRNA as well, clones in which only the active center (A₆) was substituted with guanine (A₆>G) were detected.

From these results, it was confirmed that the width of the activity window of ABE8e can be adjusted even when guanine residues are used for added nucleotide residues.

From the results of Experimental Examples 6 and 7, it was suggested that the width and position of the activity window can be freely controlled by adjusting the number and type of 5'-added nucleotide residues.

### <Experimental Example 8>

The same test as in Experimental Example 6 was performed using gRNAs of [0C] to [30C]. A base substitution test and base substitution analysis were performed in the same manner as in Experimental Example 6, except that PCR and sequence analysis were performed using the DNA of bulk cells after transfection as a template. The PCR primer sequences for amplicon sequence analysis are shown in SEQ ID NOs: 22 and 23.

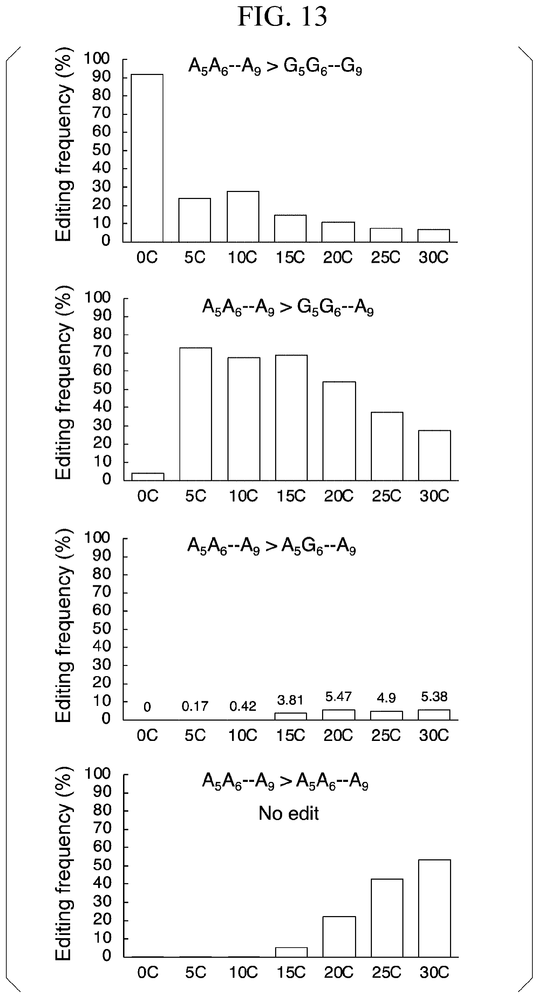

The results are shown in FIG. 13. "A₅A₆--A₉>G₅G₆--G₉" indicates the editing frequency in which all adenines (A₅, A₆, A₉) in the activity window were substituted with guanines (G₅, G₆, G₉). "A₅A₆--A₉>G₅G₆--A₉" indicates the editing frequency in which adenines (A₅, A₆) were substituted with guanines (G₅, G₆). "A₅A₆--A₉>A₅G₆--A₉" indicates the editing frequency in which A₆ was substituted with guanine (G₆). "A₅A₆-A₉>A₅A₆--A₉" indicates the editing frequency in which none of the adenines (A₅, A₆, A₉) were substituted with guanine.

With [0C] gRNA, the editing frequency of "A₅A₆--A₉>G₅G₆--G₉" was 92%. On the other hand, with gRNAs having added nucleotide residues, the editing frequency of "A₅A₆--A₉>G₅G₆--G₉" decreased significantly. The editing frequency of "A₅A₆-A₉>G₅G₆--G₉" tended to decrease as the number of added nucleotide residues increased.

With gRNAs having added nucleotide residues, the editing frequency of "A₅A₆-A₉>G₅G₆--A₉" increased significantly compared to [0C] gRNA. The editing frequency of "A₅A₆--A₉>G₅G₆--A₉" tended to decrease as the number of added nucleotide residues increased.

The editing frequency of "A₅A₆--A₉>A₅G₆--A₉" was 0% with [0C] gRNA. On the other hand, with gRNAs having added nucleotide residues, even with [5C] gRNA, the editing frequency of "A₅A₆--A₉>A₅G₆--A₉" was 0.17%. The editing frequency of "A₅A₆--A₉>A₅G₆--A₉" increased as the number of added nucleotide residues increased.

From these results, it was confirmed that the activity window of ABEe can be narrowed by adjusting the number of added nucleotide residues.

### <Experimental Example 9>

ABE8e is known to induce indels, albeit at a low rate. Therefore, the indel induction rate was investigated from the amplicon sequence analysis results of Experimental Example 8. From the amplicon sequence analysis results of [0C] gRNA, [5C] gRNA, and [10C] gRNA, the sum of the proportions of DNA having indels detected at a rate of 0.2% or more was calculated.

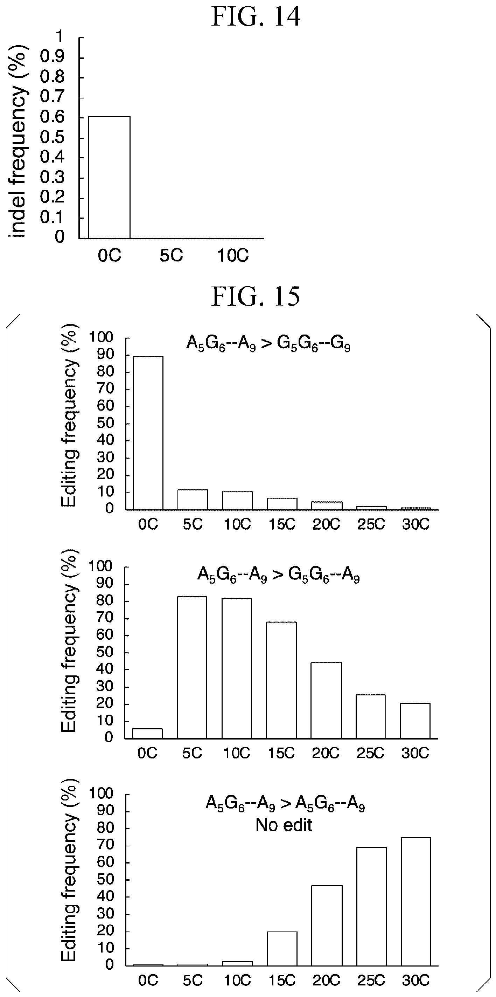

The results are shown in FIG. 14. With [0C] gRNA, indels occurred at a frequency of 0.61%. On the other hand, with [5C] gRNA and [10C] gRNA, the indel frequency was 0%. From these results, it was confirmed that indel induction by ABE8e is suppressed by added nucleotide residues.

### <Experimental Example 10>

The same test as in Experimental Example 8 was performed using the Cdh1-P2A (P2A (A₆>G))-tdTomato chimeric gene (SEQ ID NO: 30) in which A₆, the active center of the activity window of ABE8e, is pre-substituted with G. This investigated whether stepwise editing by re-editing from A₅G₆A₉ to G₅G₆G₉ is involved in the phenomenon of the wide activity window of ABE8e occurring. That is, it was investigated whether the off-target suppression effect is involved in the mechanism by which the activity window of ABE8e is narrowed by gRNA having 5'-added nucleotide residues.

A base substitution test and base substitution analysis were performed in the same manner as in Experimental Example 8, except that the P2A-3 target sequence region of the Cdh1-P2A-tdTomato chimeric gene was changed to a sequence in which A₆ is substituted with guanine (G) (P2A (A₆>G)), and the spacer sequence of gRNA was changed to P2A (A₆>G). The nucleotide sequence of the Cdh1-P2A (A₆>G)-tdTomato chimeric gene is shown in SEQ ID NO: 30. The same PCR primers for amplicon sequence analysis as in Experimental Example 8 were used.

The results are shown in FIG. 15. "A₅G₆--A₉>G₅G₆--G₉" indicates the editing frequency in which adenines (A₅, A₉) were substituted with guanines (G₅, G₉). "A₅G₆-A₉>G₅G₆--A₉" indicates the editing frequency in which adenine (A₅) was substituted with guanine (G₅). "A₅G₆--A₉>A₅G₆--A₉" indicates the editing frequency (no edit) in which none of the adenines (A₅, A₉) were substituted with guanine.

With [0C] gRNA, the editing frequency of "A₅G₆--A₉>G₅G₆--G₉" was 89%. On the other hand, with gRNAs having added nucleotide residues, the editing frequency of "A₅G₆--A₉>G₅G₆--G₉" decreased significantly. The editing frequency of "A₅G₆-A₉>G₅G₆--G₉" decreased as the number of added nucleotide residues increased.

With gRNAs having added nucleotide residues, the editing frequency of "A₅G₆-A₉>G₅G₆--A₉" increased significantly compared to [0C] gRNA. The editing frequency of "A₅A₆--A₉>G₅G₆--A₉" tended to decrease as the number of added nucleotide residues increased.

The editing frequency without substitution of "A₅G₆--A₉>A₅G₆--A₉" was 0.73% with [0C] gRNA. On the other hand, with gRNAs having added nucleotide residues, the editing frequency without substitution of "A₅G₆--A₉>A₅G₆--A₉" was 0.87% with [5C], and 2.67% with [10C]. The editing frequency without substitution of "A₅G₆-A₉>A₅G₆--A₉" increased as the number of added nucleotide residues increased.

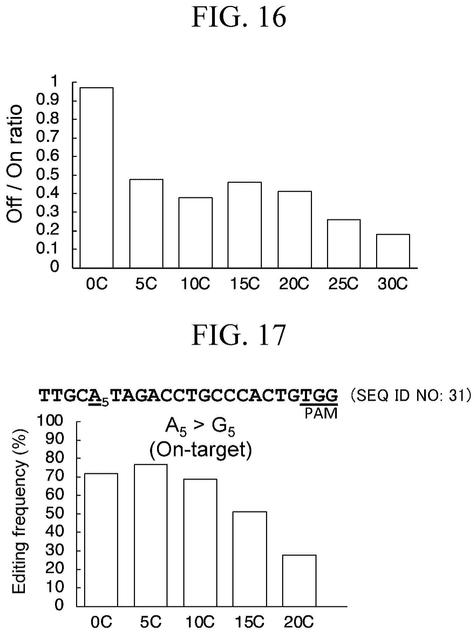

FIG. 16 shows the value (Off/On ratio) obtained by dividing the "Editing frequency (%)" of "A₅G₆--A₉>G₅G₆--G₉" in FIG. 15 by the "Editing frequency (%)" of "A₅A₆--A₉>G₅G₆--G₉" in FIG. 13. The Off/On ratio of [5C] gRNA decreased to about 1/2 compared to [0C] gRNA. The Off/On ratio tended to decrease as the number of added nucleotide residues increased.

From these results, it was confirmed that with gRNA having 5'-added nucleotide residues, re-editing by ABE8e is avoided by enabling a decrease in off-target relative to on-target, thereby narrowing the activity window.

### <Experimental Example 11>

The same test as in Experimental Example 8 was performed using HEK293T cells instead of Cdh1-AIMS. As the target sequence of gRNA, "TTGCATAGACCTGCCCACTGTGG" (SEQ ID NO: 31; "TGG" at the 3' end is the PAM sequence) was used. An ABE8e all-in-one plasmid expressing gRNA targeting the target sequence was prepared. This was transfected into HEK293T cells to induce base substitution. Using the DNA of bulk cells after transfection as a template, the target region was amplified by PCR. Then, the nucleotide sequence of the amplified DNA fragment was obtained by Sanger sequencing. The linker sequences for preparing the plasmid are shown in SEQ ID NOs: 34 and 35. The PCR primer sequences for amplicon sequence analysis for the On-target region are shown in SEQ ID NOs: 32 and 33. The PCR primer sequences for amplicon sequence analysis for the Off-target 1 (SEQ ID NO: 36) region are shown in SEQ ID NOs: 38 and 39. The PCR primer sequences for amplicon sequence analysis for the Off-target 2 (SEQ ID NO: 37) region are shown in SEQ ID NOs: 38 and 39.

The analysis results of the On-target region are shown in FIG. 17. The sequence described at the top ofFIG. 17 is the target sequence of gRNA. The editing frequency in the On-target region tended to decrease as the number of added nucleotide residues increased.

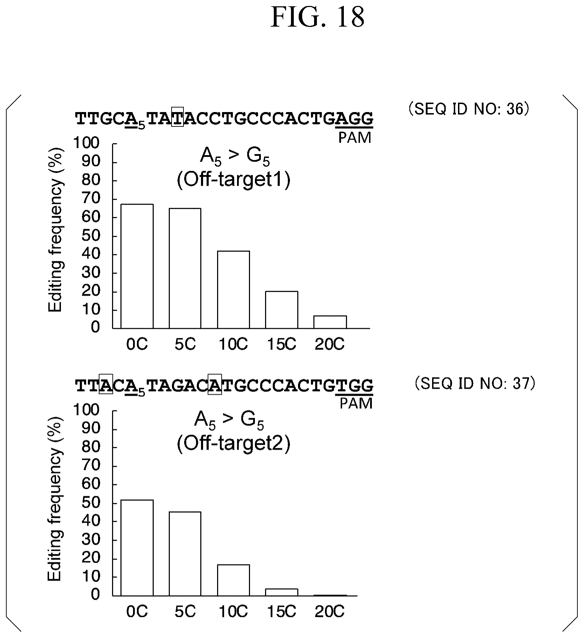

The analysis results of the Off-target regions are shown in FIG. 18. The sequences described at the top of each graph in FIG. 18 show the sequence of the Off-target 1 region and the sequence of the Off-target 2 region, respectively. The nucleotide residues enclosed in a square are mismatched residues. For both Off-target 1 and Off-target 2, the editing frequency decreased as the number of added nucleotide residues increased.

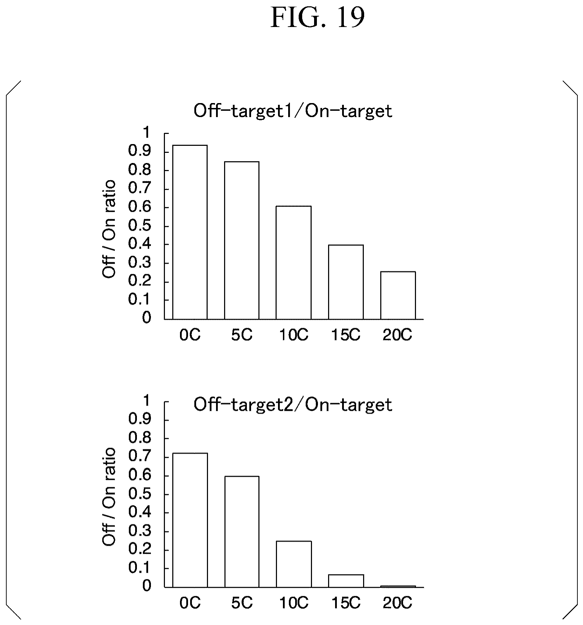

FIG. 19 shows the values (Off/On ratios) obtained by dividing the editing frequency in the Off-target regions by the editing frequency in the On-target. The upper figure uses the editing frequency of the Off-target 1 region. The lower figure uses the editing frequency of the Off-target 2 region. For both Off-target 1 and Off-target 2, the Off/On ratio decreased as the number of added nucleotide residues increased.

From these results, it was confirmed that the off-target suppression effect increases as the number of added nucleotide residues increases.

### <Experimental Example 12>

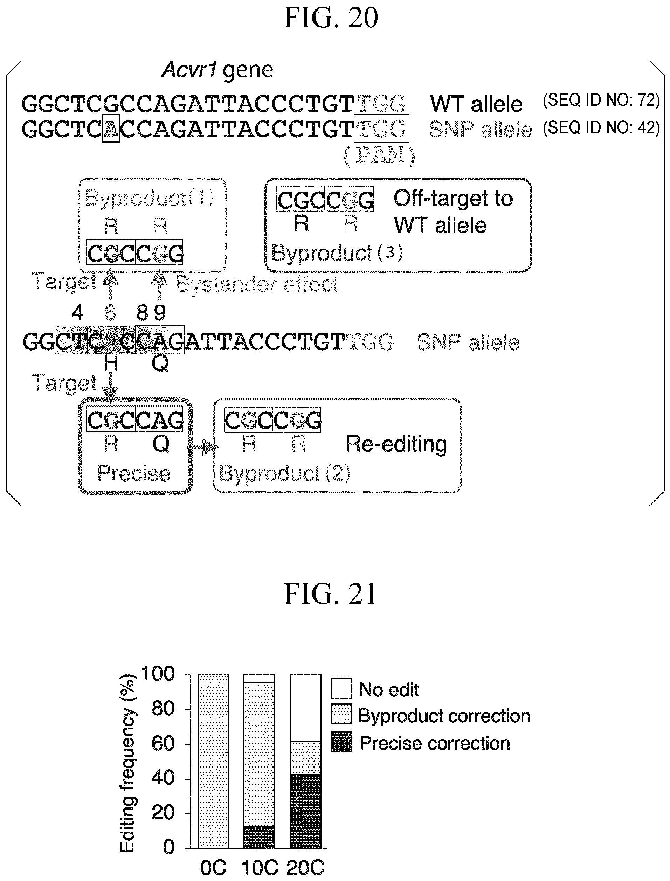

The same test as in Experimental Example 6 was performed using ES cells from a fibrodysplasia ossificans progressiva (FOP) model mouse (Kawamata et al., Nat. Biomed. Eng. 7, 672-691 (2023), PCT International Publication No. WO2020/122195) instead of Cdh1-AIMS. The FOP model mouse has a heterozygous SNP (GGCTCACCAGATAACCCTGTTGG: SEQ ID NO: 42; "TGG" at the 3' end is the PAM sequence) in the Acvrl gene (refer to FIG. 20). An ABE8e all-in-one plasmid expressing sgRNA targeting the Acvrl gene SNP was prepared. This was transfected into FOP model mouse ES cells to induce base substitution. Using the DNA of the clones obtained after transfection as a template, the Acvr1 gene SNP region was amplified by PCR. Then, the nucleotide sequence of the amplified DNA fragment was obtained by Sanger sequencing. The PCR primer sequences for Sanger sequence analysis for the Acvrl gene SNP region are shown in SEQ ID NOs: 43 and 44.

The results are shown in FIG. 21. In FIG. 21, "No edit" indicates clones that were not base-substituted. "Byproduct correction" indicates clones in which two adenines (A₆, A₉) in the Acvrl gene SNP region were substituted with guanines (G₆, G₉). "Precise correction" indicates clones in which adenine (A₆) in the Acvrl gene SNP was substituted with guanine (G₆). With [0C] gRNA, all clones were "Byproduct correction." On the other hand, with [10C] gRNA and [20C] gRNA, "Precise correction" clones were detected. The proportion of "Precise correction" was larger with [20C] gRNA than with [10C] gRNA.

As mechanisms by which "Byproduct correction" occurs, Byproducts (1) to (3) shown in FIG. 20 are conceivable. With gRNA having added nucleotide residues, since any mechanism of Byproducts (1) to (3) is suppressed, it is presumed that the proportion of "Precise correction" increases. That is, with gRNA having added nucleotide residues, it is presumed that the mechanism of Byproduct (1) is suppressed by narrowing the activity window of ABE8e. In addition, with gRNA having added nucleotide residues, it is presumed that the mechanism of Byproduct (2) is suppressed by suppressing re-editing of the allele after substitution of adenine (A₆) with guanine (G₆). Furthermore, with gRNA having added nucleotide residues, it is presumed that the mechanism of Byproduct (3) is suppressed by suppressing the off-target effect.

### <Experimental Example 13>

A base substitution test by ABE8e was performed using ES cells established from a hereditary tyrosinemia type I (HT-1)) disease model mouse (Fah^{1R/1R}). HT-1 in this mouse model is a disease developed by substitution of a guanine residue in exon 8 of the Fah gene with an adenine residue.

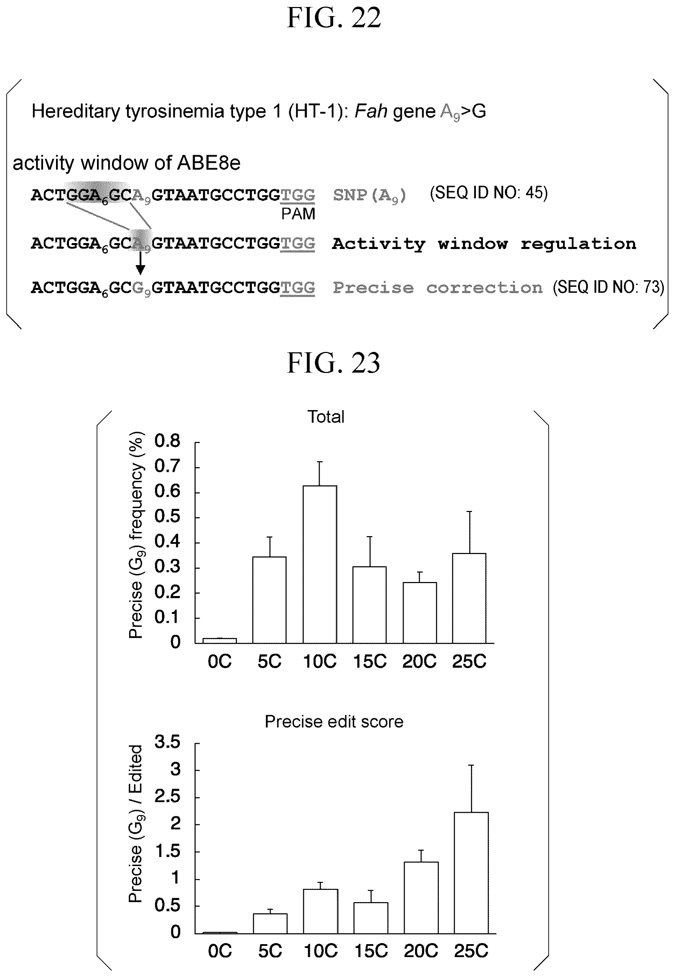

FIG. 22 shows a treatment strategy for HT-1. HT-1 can be treated by substituting the adenine residue (A₉), which is an SNP in the Fah gene, with a guanine residue (G₉) to make it a normal Fah gene. A₉ is outside the activity window of ABE8e. Within the activity window of ABE8e, there is an adenine residue (A₆) that is not an SNP. Therefore, for the treatment of HT-1, it is necessary to narrow the active window of ABE8e and shift the active window to the 3' side. It was investigated whether such precise correction can be achieved by gRNA having added nucleotide residues.

The same test as in Experimental Example 8 was performed using HT-1 model mouse ES cells instead of Cdh1-AIMS. As the target sequence of gRNA, "ACTGGAGCAGTAATGCCTGGTGG" (SEQ ID NO: 45; "TGG" at the 3' end is the PAM sequence) was used. An ABE8e all-in-one plasmid expressing gRNA targeting the target sequence was prepared. This was transfected into the HT-1 model mouse ES cells to induce base substitution. Using the DNA of bulk cells after transfection as a template, the target sequence region was amplified by PCR. Then, the nucleotide sequence of the amplified DNA fragment was obtained by Sanger sequencing. The PCR primer sequences for amplicon sequence analysis are shown in SEQ ID NOs: 46 and 47.

The results are shown in FIG. 23. The upper figure shows the precise correction frequency. With [0C] gRNA, the precise correction frequency was 0.02%. On the other hand, with gRNAs having added nucleotide residues, the precise correction frequency increased significantly. With [10C] gRNA, the precise correction frequency could be increased to 0.63%.

The lower figure in FIG. 23 shows the proportion of precise correction in alleles in which base substitution occurred. This investigated whether added nucleotide residues have the property of shifting the activity window of ABE8e to A₉. The proportion of precise correction increased as the number of added nucleotide residues increased. From these results, it was confirmed that added nucleotide residues have the effect of shifting the activity window of ABE8e. This can be said to enable pinpoint editing of A₆.

### <Experimental Example 14>

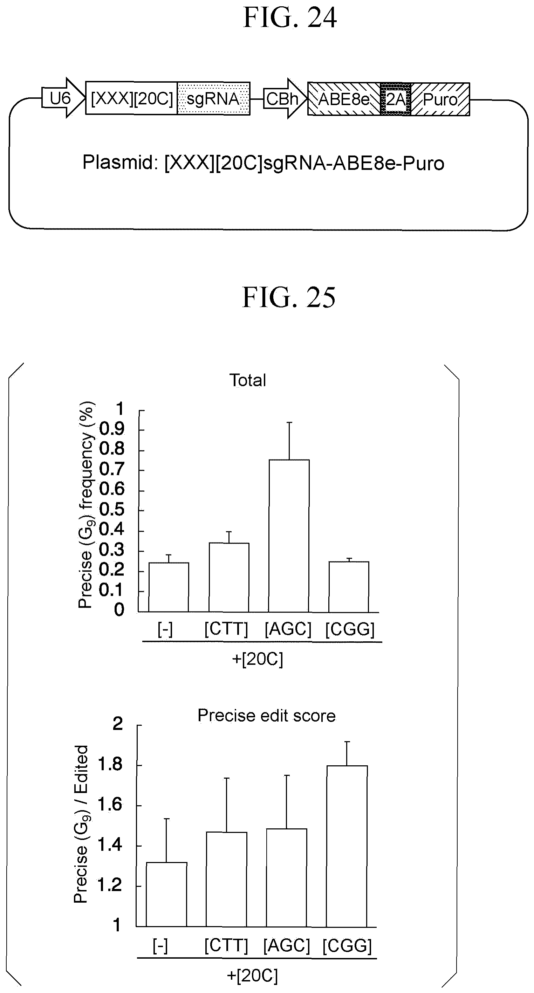

As added nucleotide residues, those in which three nucleotide residues ([XXX]) were further added to the 5' side of [20C] were used (refer to FIG. 24). As [XXX], [CTG], [AGC], or [CGG] was used. An ABE8e all-in-one plasmid expressing [XXX][20C] gRNA (Plasmid: [XXX][20C] sgRNA-ABE8e-Puro in FIG. 24) was prepared. This was transfected into HT-1 model mouse ES cells to induce base substitution. Using the DNA of bulk cells after transfection as a template, the target sequence region was amplified by PCR. Then, the nucleotide sequence of the amplified DNA fragment was obtained by amplicon sequencing. The sequences of the linkers for preparing sgRNA are shown in SEQ ID NOs: 50 to 55. The same PCR primer sequences for amplicon sequence analysis as in Experimental Example 13 were used.

The results are shown in FIG. 25. The upper figure shows the precise correction frequency. By further adding three nucleotide residues to the 5' side of [20C], the precise correction frequency changed. With [AGC][20C] gRNA, the precise correction frequency increased by 3.1 times compared to [20C] gRNA.

The lower figure in FIG. 25 shows the proportion of precise correction in alleles in which base substitution occurred. By further adding three nucleotide residues to [20C], the proportion of precise correction increased compared to [20C] gRNA.

From these results, it is presumed that by adjusting the number and sequence of added nucleotide residues, the activity window of ABE8e can be narrowed and shifted to a desired position. This is expected to enable repair of SNPs that could not originally be targeted, expanding the number of diseases that can be treated.

### <Experimental Example 15>

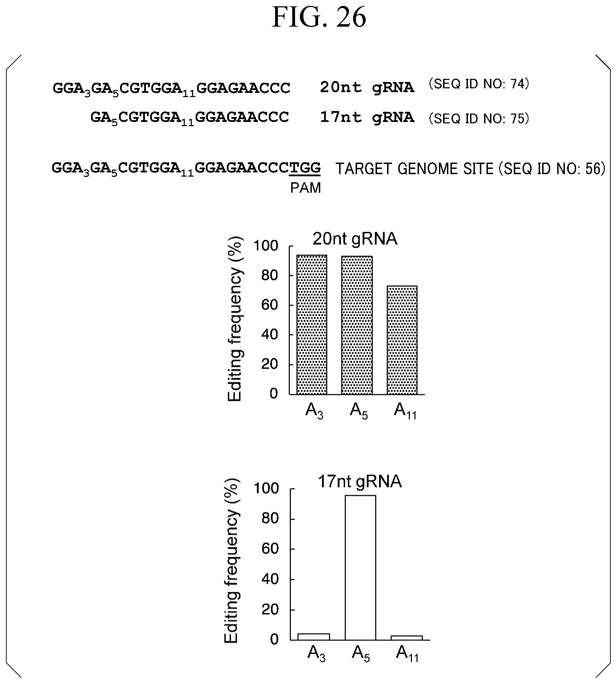

The same test as in Experimental Example 6 was performed using P2A-8 (GGAGACGTGGAGGAGAACCCTGG: SEQ ID NO: 56; "TGG" at the 3' end is the PAM sequence) as the target sequence. As the spacers of sgRNA, a 20 nt spacer (SEQ ID NO: 74) and a 17 nt spacer (SEQ ID NO: 75) were used (refer to FIG. 26). The linker sequences for preparing sgRNA with the 20 nt spacer (20 nt gRNA) are shown in SEQ ID NOs: 57 and 58. The linker sequences for preparing sgRNA with the 17 nt spacer (17 nt gRNA) are shown in SEQ ID NOs: 59 and 60.

A base substitution test and base substitution analysis were performed in the same manner as in Experimental Example 8, except that an ABE8e all-in-one plasmid expressing 20 nt gRNA or 17 nt gRNA was used. The same PCR primers for amplicon sequence analysis as in Experimental Example 8 were used.

The results are shown in FIG. 26. With the 17 nt gRNA, the bystander effect at A₃ and A₁₁ was strongly suppressed, and the editing frequency of base substitution at A₅ increased significantly. From these results, it was confirmed that the activity window can be narrowed by adjusting the length of the spacers. This is considered to enable precise genome editing that avoids the bystander effect.

### <Experimental example 16>

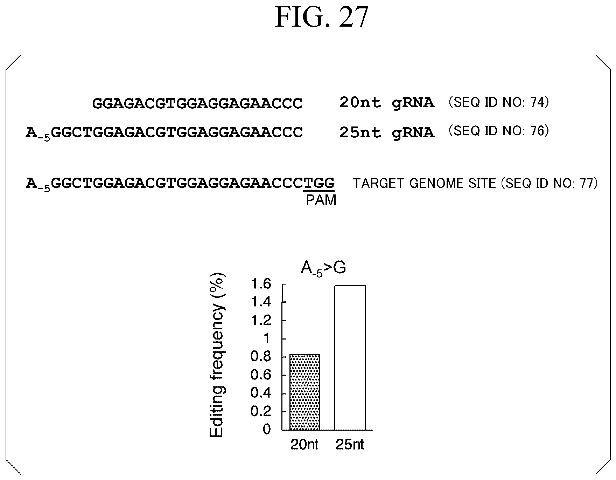

A base substitution test and base substitution analysis were performed in the same manner as in Experimental Example 15, except that a 20 nt spacer (SEQ ID NO: 74) and a 25 nt spacer (SEQ ID NO: 76) were used as the spacers of sgRNA (refer to FIG. 27). The linker sequences for preparing sgRNA with the 25 nt spacer (25 nt gRNA) are shown in SEQ ID NOs: 61 and 62. The same PCR primers for amplicon sequence analysis as in Experimental Example 8 were used.

The results are shown in FIG. 27. With the 25 nt gRNA, the editing frequency at A₋₅ increased by 1.9 times compared to the 20 nt gRNA. From these results, it was confirmed that the activity window can be shifted to the 5' side by increasing the length of the spacers. It is considered that by enabling editing for portions where base substitution cannot be thoroughly performed due to the restriction of the PAM sequence, the range of practicality as a genome editing tool will expand.

### <Experimental example 17>

A base substitution test and base substitution analysis were performed in the same manner as in Experimental Example 15, except that AID-BE4max (cytidine base editor) was used instead of ABE8e.

The AID all-in-one plasmid (SEQ ID NO: 63) was prepared by the following method. From the AID-BE4max-P2A-GFP plasmid (Addgene, Plasmid #157948) (SEQ ID NO: 64), the region including the NLS-P2A-GFP-NLS cassette was excised using EcoRI and PmeI. Then, the T2A-Puro cassette (SEQ ID NO: 65) of the pSpCas9(BB)-2A-Puro (PX459) V2.0 plasmid (SEQ ID NO: 4) was ligated to the EcoRI and PmeI cleavage sites. This allowed preparation of the AID all-in-one plasmid.

The T2A-Puro cassette (SEQ ID NO: 65) was obtained as follows. PCR was performed using the pSpCas9(BB)-2A-Puro (PX459) V2.0 plasmid as a template. At that time, an EcoRI site and a nuclear localization signal (NLS) were added to the 5' end of the forward primer (SEQ ID NO: 66), and the stop codon (TGA) of the Puro cassette and a PmeI site were added to the 3' end of the reverse primer (SEQ ID NO: 67).

P2A-8 (SEQ ID NO: 56) was used as the target sequence. The same linkers for preparing 20 nt gRNA, linkers for preparing 17 nt gRNA, and PCR primers for amplicon sequence analysis as in Experimental Example 15 were used.

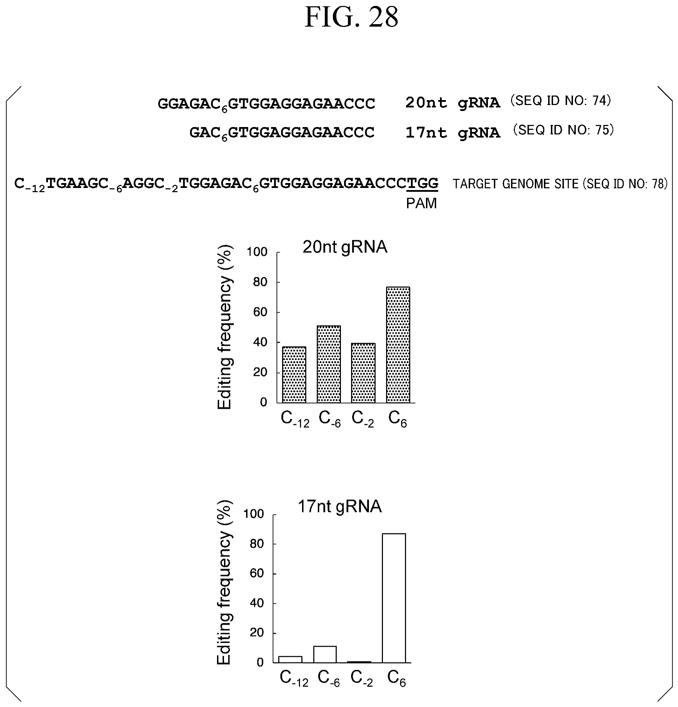

The results are shown in FIG. 28. With the 17 nt gRNA, the bystander effect at C₋₁₂, C₋₆, and C₋₂ was strongly suppressed, and the editing frequency at C₆ increased. An effect of narrowing the activity window of AID-BE4max was obtained. From these results, it was confirmed that the active window can be narrowed by adjusting the length of the spacer.

### <Experimental example 18>

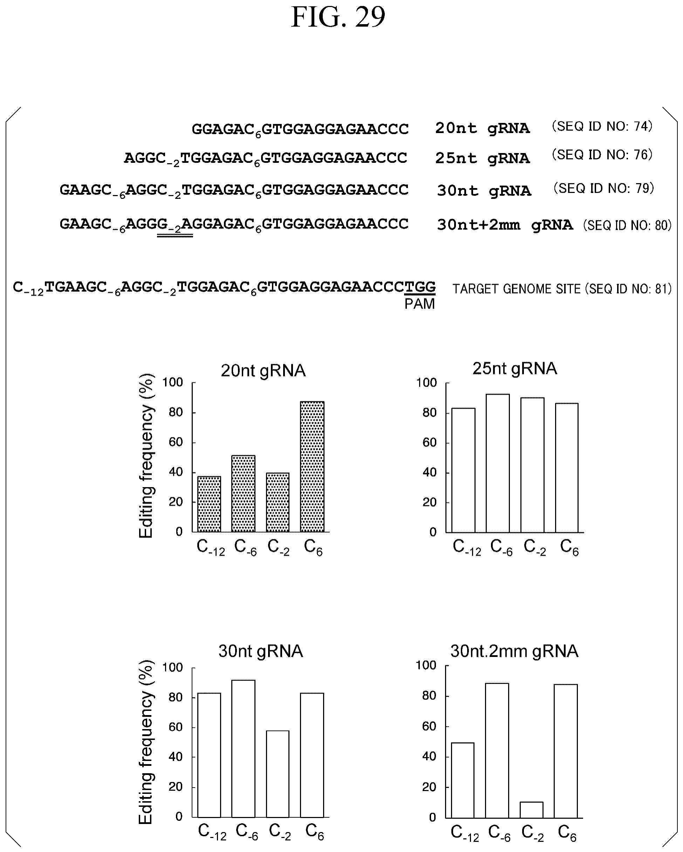

As the spacers of sgRNA, a 20 nt spacer (SEQ ID NO: 74), a 25 nt spacer, a 30 nt spacer, and a 30 nt + 2 mismatch spacer (having 2 mismatches in the 30 nt spacer sequence) were used (refer to FIG. 29). The linker sequences for preparing sgRNA with the 20 nt spacer (20 nt gRNA) are shown in SEQ ID NOs: 57 and 58. The linker sequences for preparing sgRNA with the 25 nt spacer (25 nt gRNA) are shown in SEQ ID NOs: 61 and 62. The linker sequences for preparing sgRNA with the 30 nt spacer (30 nt gRNA) are shown in SEQ ID NOs: 68 and 69. The linker sequences for preparing sgRNA with the 30 nt + 2 mismatch spacer (30 nt + 2 mm gRNA) are shown in SEQ ID NOs: 70 and 71.

A base substitution test and base substitution analysis were performed in the same manner as in Experimental Example 17, except that an AID all-in-one plasmid expressing 20 nt gRNA, 25 nt gRNA, 30 nt gRNA, or 30 nt + 2 mm gRNA was used. The same PCR primers for amplicon sequence analysis as in Experimental Example 17 were used.

The results are shown in FIG. 29. With the 25 nt gRNA and 30 nt gRNA, the editing frequency at C₋₁₂, C₋₆, and C₋₂ increased compared to the 20 nt gRNA. From these results, it was confirmed that the effect of expanding the activity window of AID-BE4max to the 5' side can be obtained by extending the spacer to the 5' side. With the 30 nt + 2 mm gRNA, the editing efficiency at C₋₆ and C₆ was comparable to the 30 nt gRNA, but the editing frequency at C₋₂ decreased significantly. In the 30 nt + 2 mm gRNA, the nucleotide residue corresponding to C₋₂ of the target genome site is converted to a guanine residue (G). The decrease in editing frequency at C₋₂ is considered to be caused by this mismatch. From these results, it was confirmed that by introducing mismatches into the extension portion to the 5' side of the spacer, the activity window can be expanded to the 5' side while avoiding substitution of arbitrary bases. This is considered to enable base substitution designs that precisely repair specific disease SNPs.

The sequences used in Experimental Examples 1 to 5 are shown in Tables 1 to 7. In the tables, "F" represents forward. "R" represents reverse.

**[Table 1]**

| SEQ ID NO | Gene | Application | Direction |
|---|---|---|---|
| 82 | ASCL1 (CRISPRa) | q-PCR | F |
| 83 | | | R |
| 84 | TTN (CRISPRa) | | F |
| 85 | | | R |
| 86 | BRCA1 (CRISPRi) | | F |
| 87 | | | R |
| 88 | CXCR4 (CRISPRi) | | F |
| 89 | | | R |
| 90 | GAPDH | | F |
| 91 | | | R |

**[Table 2]**

| For [C]sgRNA PX459 plasmids | | | |
|---|---|---|---|
| SEQ ID NO | sgRNA name | Extension | Direction |
| 92 | P2A1-sgRNA1 | [15C] | F |
| 93 | | | R |
| 94 | | [15G] | F |
| 95 | | | R |
| 96 | | [15A] | F |
| 97 | | | R |
| 98 | | [15T] | F |
| 99 | | | R |
| 100 | | [0C] | F |
| 101 | | | R |
| 102 | | [5C] | F |
| 103 | | | R |
| 104 | | [10C] | F |
| 105 | | | R |
| 106 | | [15C] | F |
| 107 | | | R |
| 108 | | [20C] | F |
| 109 | | | R |
| 110 | | [25C] | F |
| 111 | | | R |
| 112 | | [30C] | F |
| 113 | | | R |
| 114 | | [15hp] | F |
| 115 | | | R |
| 116 | | [15s] | F |
| 117 | | | R |

**[Table 3]**

| SEQ ID NO | Application | Direction |
|---|---|---|
| 118 | mU6 probe for Northern blot (93 bp) | F |
| 119 | | R |
| 120 | Priming for mU cDNA synthesis | R |
| 121 | Probe for Northern blot | F |
| 122 | | R |

**[Table 4]**

| CRISPRa | | | |
|---|---|---|---|
| SEQ ID NO | sgRNA name | Extension | Direction |
| 123 | ASCL1-gRNA | [0C] | F |
| 124 | | | R |
| 125 | | [1C] | F |
| 126 | | | R |
| 127 | | [2C] | F |
| 128 | | | R |
| 129 | | [3C] | F |
| 130 | | | R |
| 131 | | [4C] | F |
| 132 | | | R |
| 133 | | [5C] | F |
| 134 | | | R |
| 135 | | [6C] | F |
| 136 | | | R |
| 137 | | [10C] | F |
| 138 | | | R |
| 139 | TTN-gRNA | | F |
| 140 | | | R |

**[Table 5]**

| CRISPRa | | | |
|---|---|---|---|
| SEQ ID NO | sgRNA name | Extension | Direction |
| 141 | BRCA1-gRNA | [0C] | F |
| 142 | | | R |
| 143 | | [1C] | F |
| 144 | | | R |
| 145 | | [2C] | F |
| 146 | | | R |
| 147 | | [3C] | F |
| 148 | | | R |
| 149 | | [4C] | F |
| 150 | | | R |
| 151 | | [5C] | F |
| 152 | | | R |
| 153 | | [6C] | F |
| 154 | | | R |
| 155 | | [7C] | F |
| 156 | | | R |
| 157 | CXCR4-gRNA | | F |
| 158 | | | R |

**[Table 6]**

| SEQ ID NO | Name | Type |
|---|---|---|
| 159 | VP64-T2A-PuroR | Synthetic DNA |
| 160 | U6-crRNA cloning cassettes (AsCpfl) | Synthetic DNA |

**[Table 7]**

| AsCpf1 (P2A2-gRNA7) | | | |
|---|---|---|---|
| SEQ ID NO | Location | Extension | Direction |
| 161 | 5' end | [0C] | F |
| 162 | | | R |
| 163 | | [5C] | F |
| 164 | | | R |
| 165 | | [10C] | F |
| 166 | | | R |
| 167 | | [15C] | F |
| 168 | | | R |
| 169 | | [20C] | F |
| 170 | | | R |
| 171 | | [25C] | F |
| 172 | | | R |
| 173 | | [30C] | F |
| 174 | | | R |
| 175 | middle | [10C] | F |
| 176 | | | R |
| 177 | | [25C] | F |
| 178 | | | R |
| 179 | 3' end | [10C] | F |
| 180 | | | R |
| 181 | | [25C] | F |
| 182 | | | R |

The sequences used in Experimental Examples 6 to 18 are shown in Tables 8 to 11.

**[Table 8]**

| SEQ ID NO | Sequence name | Description of sequence |
|---|---|---|
| 1 | Target P2A-3 | Target sequence of P2A-3 |
| 2 | ABE8e all-in-one (p.KW955-1) | ABE8e all-in-one plasmid: PX459 plasmid backbone |
| 3 | ABE8e | ABE8e expression vector |
| 4 | pSpCas9(BB)-2A-Puro (PX459) V2.0 | CRISPR all-in-one plasmid |
| 5 | Pr. KW1185 | Linker (F) for construction of sgRNA(SC) expression ABE8e all-in-one vector |
| 6 | Pr. KW1186 | Linker (R) for construction of sgRNA(SC) expression ABE8e all-in-one vector |
| 7 | Pr. KW864 | Linker (F) for construction of sgRNA(10C) expression ABE8e all-in-one vector |
| 8 | Pr. KW865 | Linker (R) for construction of sgRNA(10C) expression ABE8e all-in-one vector |
| 9 | Pr. KW1187 | Linker (F) for construction of sgRNA(15C) expression ABE8c all-in-one vector |
| 10 | Pr. KW1188 | Linker (R) for construction of sgRNA(15C) expression ABE8e all-in-one vector |
| 11 | Pr. KW1189 | Linker (F) for construction of sgRNA(20C) expression ABE8c all-in-one vector |
| 12 | Pr. KW1190 | Linker (R) for construction of sgRNA(20C) expression ABE8e all-in-one vector |
| 13 | Pr. KW866 | Linker (F) for construction of sgRNA(25C) expression ABE8e all-in-one vector |
| 14 | Pr. KW867 | Linker (R) for construction of sgRNA(25C) expression ABE8e all-in-one vector |
| 15 | Pr. KW1191 | Linker (F) for construction of sgRNA(30C) expression ABE8e all-in-one vector |
| 16 | Pr. KW1192 | Linker (R) for construction of sgRNA(30C) expression ABE8e all-in-one vector |
| 17 | Pr. KW950 | Linker (F) for construction of P2A-sgRNA3 expression vector |
| 18 | Pr. KW951 | Linker (R) for construction of P2A-sgRNA3 expression vector |
| 19 | Cdh1-AIMS-tdTomato allele sequence | Sequence for sequence analysis of Cdh1 -AIMS-tdTomato allele |
| 20 | Pr. KW1117 | Primer (F) for PCR of Cdh1-AIMS-tdTomato allele (Sanger sequencing) |

**[Table 9]**

| SEQ ID NO | Sequence name | Description of sequence |
|---|---|---|
| 21 | Pr. KW552 | Primer (R) for PCR of Cdh1-AIMS-tdTomato allele (Sanger sequencing) |
| 22 | Pr. KW1117 (NGS) | Primer (F) for PCR of Cdh1-AIMS-tdTomato allele (amplicon sequencing) |
| 23 | Pr. KW552 (NGS) | Primer (R) for PCR of Cdh1-AIMS-tdTomato allele (amplicon sequencing) |
| 24 | Pr. KW1869 | Linker (F) for construction of sgRNA(30G) expression ABE8e all-in-one vector |
| 25 | Pr. KW1870 | Linker (R) for construction of sgRNA(30G) expression ABE8e all-in-one vector |
| 26 | Pr. KW1871 | Linker (F) for construction of sgRNA(35G) expression ABE8e all-in-one vector |
| 27 | Pr. KW1872 | Linker (R) for construction of sgRNA(35G) expression ABE8e all-in-one vector |
| 28 | Pr. KW1873 | Linker (F) for construction of sgRNA(40G) expression ABE8e all-in-one vector |
| 29 | Pr. KW1874 | Linker (R) for construction of sgRNA(40G) expression ABE8e all-in-one vector |
| 30 | P2A (A6>G)-tdTomato | Cells in which A6 of target sequence of P2A-3 on P2A (A6>G)-tdTomato chimeric gene side is substituted with G |
| 31 | On-target in HEK293T | On-target target sequence in HEK293T |
| 32 | Pr. KW2236 (NGS) | Primer (F) for PCR of On-target in HEK293T (amplicon sequencing) |
| 33 | Pr. KW2237 (NGS) | Primer (R) for PCR of On-target in HEK293T (amplicon sequencing) |
| 34 | Pr. KW2232 | Linker (F) for construction of HEK293T On-target expression vector |
| 35 | Pr. KW2233 | Linker (R) for construction of HEK293T On-target expression vector |
| 36 | Off-target 1 in HEK293T | Off-target 1 target sequence in HEK293T |
| 37 | Off-target 1 in HEK293T | Off-target 2 target sequence in HEK293T |
| 38 | Pr. KW2238 (NGS) | Primer (F) for PCR of Off-target 1 in HEK293T (amplicon sequencing) |
| 39 | Pr. KW2239 (NGS) | Primer (R) for PCR of Off-target 1 in HEK293T (amplicon sequencing) |
| 40 | Pr. KW2240 (NGS) | Primer (F) for PCR of Off-target 2 in HEK293T (amplicon sequencing) |

**[Table 10]**

| SEQ ID NO | Sequence name | Description of sequence |
|---|---|---|
| 41 | Pr. KW2241 (NGS) | Primer (R) for PCR of Off-target 2 in HEK293T (amplicon sequencing) |
| 42 | FOP SNP allele sequence | Target sequence of SNP allele in FOP model mouse ES cells |
| 43 | Pr. KW1201 | Primer (F) for PCR of FOP allele (Sanger sequencing) |
| 44 | Pr. KW1202 | Primer (R) for PCR of FOP allele (Sanger sequencing) |
| 45 | TH-1 Fah SNP allele sequence | Target sequence of SNP allele in TH-1 Fah1R/1R mouse model ES cells |
| 46 | Pr. KW1232 (NGS) | Primer (F) for PCR of TH-1 Fah1R/1R (Sanger sequencing) |
| 47 | Pr. KW2080 (NGS) | Primer (R) for PCR of TH-1 Fah1R/1R (Sanger sequencing) |
| 48 | Pr. KW1327 | Linker (F) for construction of TH-1 Fah1R/1R SNP-target expression vector |
| 49 | Pr. KW1328 | Linker (R) for construction of TH-1 Fah1R/1R SNP-target expression vector |
| 50 | Pr. TK54 | Linker (F) for construction of TH-1 Fah1R/1R SNP-target expression vector: [CTT][20C] |
| 51 | Pr. TK119 | Linker (R) for construction of TH-1 Fah1R/1R SNP-target expression vector: [CTT] [20C] |
| 52 | Pr. TK12 | Linker (F) for construction of TH-1 Fah1R/1R SNP-target expression vector: [AGC][20C] |
| 53 | Pr. TK77 | Linker (R) for construction of TH-1 Fah1R/1R SNP-target expression vector: [AGC][20C] |
| 54 | Pr. TK59 | Linker (F) for construction of TH-1 Fah1R/1R SNP-target expression vector: [CGG][20C] |
| 55 | Pr. TK124 | Linker (R) for construction of TH-1 Fah1R/1R SNP-target expression vector: [CGG][20C] |
| 56 | Target P2A-8 | Target sequence of P2A-8 |
| 57 | Pr. KW2216 | Linker (F) for construction of P2A-sgRNA8 expression vector: 20 nt |
| 58 | Pr. KW2217 | Linker (R) for construction of P2A-sgRNA8 expression vector: 20 nt |
| 59 | Pr. KW2224 | Linker (F) for construction of P2A-sgRNA8 expression vector: 17 nt |
| 60 | Pr. KW2225 | Linker (R) for construction of P2A-sgRNA8 expression vector: 17 nt |

**[Table 11]**

| SEQ ID NO | Sequence name | Description of sequence |
|---|---|---|
| 61 | Pr. KW2218 | Linker (F) for construction of P2A-sgRNA8 expression vector: 25 nt |
| 62 | Pr. KW2219 | Linker (R) for construction of P2A-sgRNA8 expression vector: 25 nt |
| 63 | AID all-in-one (p. KW1285-1) | AID all-in-one plasmid |
| 64 | AID-BE4max-P2A-GFP | AID-BE4max-P2A-GFP plasmid (Addgene, Plasmid #157948) |
| 65 | T2A-Puro cassette | Preparation of T2A-Puro cassette for AID all-in-one plasmid |
| 66 | Pr. KW2210 | Primer (F) for T2A-Puro generation |
| 67 | Pr. KW2211 | Primer (R) for T2A-Puro generation |
| 68 | Pr. KW2220 | Linker (F) for construction of P2A-sgRNA8 expression vector: 30 nt |
| 69 | Pr. KW2221 | Linker (R) for construction of P2A-sgRNA8 expression vector: 30 nt |
| 70 | Pr. KW2226 | Linker (F) for construction of P2A-sgRNA8 expression vector: 30 nt. 2 mm |
| 71 | Pr. KW2227 | Linker (R) for construction of P2A-sgRNA8 expression vector: 30 nt. 2 mm |
| 72 | FOP WT | WT allele sequence of Acvr1 |
| 73 | TH-1 Fah WT | WT allele sequence of TH-1 Fah |
| 74 | P2A-8 20 nt | P2A-8 20 nt spacer sequence |
| 75 | P2A-8 17 nt | P2A-8 17 nt spacer sequence |
| 76 | P2A-8 25 nt | P2A-8 25 nt spacer sequence |
| 77 | Target P2A-8_27 | Target genome site of FIG. 27 |
| 78 | Target P2A-8_28 | Target genome site of FIG. 28 |
| 79 | P2A-8 30 nt | P2A-8 30 nt spacer sequence |
| 80 | P2A-8 30 nt + 2 mm | P2A-8 30 nt + 2 mm spacer sequence |
| 81 | Target P2A-8_29 | Target genome site of FIG. 29 |

### INDUSTRIAL APPLICABILITY

According to the present disclosure, it is possible to provide novel methods relating to a method for controlling transcription of RNA and a method for designing an expression vector for RNA, as well as novel methods applicable to application technologies of RNA-guided nucleases. In addition, it is possible to provide guide RNA, expression vectors, kits, and the like usable in the above-described methods. These can be applied to the treatment or prevention of hereditary diseases, diseases caused by abnormal gene expression, and the like. In addition, these can also be applied to a wide range of industrial fields, such as drug discovery screening, disease model preparation, food development such as functional foods, and livestock improvement.

Preferred examples of the present invention have described above, but the present invention is not limited to the examples. Additions, omissions, replacements and other modifications of the configurations can be made within a scope not departing from the gist of the present invention. The present invention is not limited by the above-described description, and is limited only by the scope of the accompanied claims.

## Claims

1. A method for controlling transcription of RNA, comprising:
a step of transcribing RNA from an expression vector for the RNA,
wherein the RNA is RNA to which one or more nucleotide residues are added at the 5' end.

2. The method for controlling transcription of RNA according to claim 1,
wherein the number of the added nucleotide residues is three or more.

3. The method for controlling transcription of RNA according to claim 1 or 2,
wherein the RNA is guide RNA.

4. A method for designing an expression vector for RNA, comprising:
designing an expression vector for RNA in which transcription efficiency of the RNA is controlled by adjusting one or more selected from the group consisting of the number of nucleotide residues to be added to the 5' end of an RNA coding sequence and the type of the nucleotide residues.

5. The method for designing an expression vector for RNA according to claim 4,
wherein the RNA is guide RNA.

6. A method for controlling transcription of a target gene, comprising:
a step of introducing into a cell
(A) at least one selected from the group consisting of (a1) guide RNA to which one or more nucleotide residues are added at the 5' end, and (a2) an expression vector for the guide RNA of (a1); and
(B) at least one selected from the group consisting of a fusion protein including an RNA-guided nuclease with inactivated nuclease activity and a transcription regulation-related domain, mRNA of the fusion protein, and an expression vector for the fusion protein,
wherein the guide RNA targets a transcription regulation region of the target gene.

7. The method for controlling transcription of a target gene according to claim 6,
wherein the number of the added nucleotide residues is three or more.

8. A genome editing method, comprising:
a step of introducing into a cell
(A) at least one selected from the group consisting of (a1) guide RNA which has a repeat sequence on the 5' side of a spacer sequence and to which one or more nucleotide residues are added at the 5' end, and (a2) an expression vector for the guide RNA of (a1); and
(B) at least one selected from the group consisting of an RNA-guided nuclease, mRNA of the RNA-guided nuclease, and an expression vector for the RNA-guided nuclease.

9. The genome editing method according to claim 8,
wherein the number of the added nucleotide residues is three or more.

10. The genome editing method according to claim 8 or 9, wherein the RNA-guided nuclease is Cas protein of a Type V CRISPR/Cas system.

11. A base editing method, comprising:
a step of introducing into a cell
(A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (a1); and
(B) at least one selected from the group consisting of a base editor, mRNA of the base editor, and an expression vector for the base editor.

12. The base editing method according to claim 11, wherein an activity window of the base editor is controlled by adjusting one or more selected from the group consisting of (1) the addition of nucleotide residues to the 5' end, (2) the length of a spacer sequence, and (3) the introduction of mismatched nucleotide residues in the spacer sequence.

13. The base editing method according to claim 12, wherein the activity window of the base editor is controlled by adjusting the addition of nucleotide residues to the 5' end, and the number of the added nucleotide residues is three or more.

14. The base editing method according to claim 12, wherein the activity window of the base editor is controlled by adjusting the length of a spacer sequence.

15. The base editing method according to claim 12, wherein the activity window of the base editor is controlled by adjusting the introduction of mismatched nucleotide residues in the spacer sequence.

16. A kit for controlling transcription of a target gene, comprising:
at least one selected from the group consisting of (a1) guide RNA to which one or more nucleotide residues are added at the 5' end, and (a2) an expression vector for the guide RNA of (a1); and
(B) at least one selected from the group consisting of a fusion protein including an RNA-guided nuclease with inactivated nuclease activity and a transcription regulation-related domain, mRNA of the fusion protein, and an expression vector for the fusion protein,
wherein the guide RNA targets a transcription regulation region of the target gene.

17. A base editing kit, comprising:
(A) at least one selected from the group consisting of (a1) guide RNA in which one or more selected from the group consisting of (1) addition of nucleotide residues to the 5' end, (2) length of a spacer sequence, and (3) introduction of mismatched nucleotide residues in the spacer sequence are adjusted, and (a2) an expression vector for the guide RNA of (a1); and
(B) at least one selected from the group consisting of a base editor, mRNA of the base editor, and an expression vector for the base editor.

18. An expression vector capable of expressing:
(A) guide RNA to which one or more nucleotide residues are added at the 5' end; and
(B) a fusion protein including an RNA-guided nuclease with inactivated nuclease activity and a transcription regulation-related protein.

19. An expression vector capable of expressing:
(A) guide RNA to which one or more nucleotide residues are added at the 5' end; and
(B) a base editor.

20. Guide RNA which has a repeat sequence on the 5' side of a spacer sequence and to which one or more nucleotide residues are added at the 5' end.

21. An expression vector capable of expressing the guide RNA according to claim 20.

22. The expression vector according to claim 21, further capable of expressing an RNA-guided nuclease.

23. A genome editing kit, comprising:
(A) at least one selected from the group consisting of (a1) guide RNA which has a repeat sequence on the 5' side of a spacer sequence and to which one or more nucleotide residues are added at the 5' end, and (a2) an expression vector for the guide RNA of (a1); and
(B) at least one selected from the group consisting of an RNA-guided nuclease, mRNA of the RNA-guided nuclease, and an expression vector for the RNA-guided nuclease.
